# EUROPÄISCHE PATENTSCHRIFT

(11) **EP 3 851 050 B1**
(45) Veröffentlichungstag und Bekanntmachung des Hinweises auf die Patenterteilung: **16.07.2025**
(21) Anmeldenummer: 20152164.8
(22) Anmeldetag: 16.01.2020
(51) Int. Cl.: A61B 6/10, A61B 6/00, A61B 8/00, A61B 34/30

(54) **MOBILE PLATFORM**
MOBILE PLATFORM
PLATEFORME MOBILE

(43) Veröffentlichungstag der Anmeldung: 21.07.2021
(73) Patentinhaber: Siemens Healthineers AG, 91301 Forchheim (DE)
(72) Erfinder: Dirauf, Franz, 96231 Bad Staffelstein (DE); Garcia, Elmar, 90427 Nürnberg (DE); Hofmann, Jörg, 91301 Forchheim (DE)
(74) Vertreter: Siemens Healthineers Patent Attorneys

(56) Entgegenhaltungen:
- EP-A1- 2 380 496
- WO-A1-2019/228530
- CN-A- 109 288 541
- US-A1- 2018 031 377
- US-A1- 2018 177 523

## Beschreibung

Die Erfindung betrifft eine mobile Plattform und ein System umfassend eine Mehrzahl von erfindungsgemäßen mobilen Plattformen.

Medizinische Geräte für Diagnose und Therapie sind in der Regel stationär angeordnet und erfordern häufig einen hohen Installationsaufwand sowie eine spezielle Gebäude-Infrastruktur. Zudem ist es häufig notwendig, dass ein Patient eine bestimmte Position einnimmt, um mit dem medizinischen Gerät untersucht oder behandelt zu werden. Beispielsweise muss sich der Patient für eine Thorax-Röntgen-Aufnahme mit dem Brustkorb direkt vor einen an einem Stativ angebrachten Röntgendetektor bzw. Detektor stellen. In manchen Fällen kann die Position des Detektors an die Größe des Patienten angepasst werden. Wenn der Patient allerdings im Rollstuhl sitzt, ist die geeignete Positionierung des Patienten erschwert. Außerdem verursacht der Transport von medizinischen Geräten und Material innerhalb eines Krankenhauses oder einer Praxis zusätzlichen Arbeitsaufwand für das medizinische Personal. Aus diesem Grund werden im medizinischen Umfeld neben stationären Geräten zunehmend auch mobile medizinische Geräte eingesetzt. Diese mobilen medizinischen Geräte sind typischerweise bewegbar und/oder verfahrbar ausgebildet. Solche mobilen medizinischen Geräte können nach Bedarf an verschiedenen Orten eingesetzt, bei Nichtgebrauch temporär aus ihrem Arbeitsumfeld entfernt und an einem geeigneten Ort geparkt werden. Beispielsweise werden für den Transport von Patienten im Klinikbetrieb mobile Patientenliegen bzw. Patientenbetten eingesetzt. Insbesondere können die Patientenliegen medizinischer Bildgebungsgeräte selbst mobil ausgebildet sein, wodurch der Arbeitsablauf vereinfacht werden kann. Weiterhin können medizinische Bildgebungsgeräte mobil ausgebildet sein, wobei insbesondere ihre Gantry mobil ausgebildet sein kann. Insbesondere sind mobile Röntgensysteme, mobile Befundungsstationen, mobile Geräte für die Intensivmedizin und mobile Robotersysteme für medizinische Anwendungen bekannt.

So offenbart die EP 2 380 496 A1 eine mobile Plattform, umfassend ein Fahrwerk, ein Sensormodul und eine Haltevorrichtung in Form eines C-Arms. Das Fahrwerk und/oder die eine Haltevorrichtung sind ausgebildet, die Arbeitsposition des an der mindestens einen Haltevorrichtung angebrachten ersten medizinischen Gerätes der Bewegung eines zweiten medizinischen Gerätes anzupassen, wobei das zweite medizinische Gerät, ein Patiententisch, von einer zweiten mobilen Plattform oder von einer immobilen Plattform geführt wird.

Allerdings bieten herkömmliche mobil einsetzbare medinische Geräte üblicherweise nur eingeschränkte Funktionalität und arbeiten weniger effizient als stationäre Geräte.

Es ist bekannt, für die medizinische Bildgebung, insbesondere für die radiologische und nuklearmedizinische Bildgebung, Halte- und/oder Führungssysteme, wie z.B. boden-, decken oder wandgebundene Schienensysteme und/oder Haltestrukturen sowie Roboterarme etc. zu verwenden (z.B. Radiographie-, Angiographie-Systeme etc.). Alternativ werden bildgebende Systeme mit starrer geometrischer Anordnung der Systemmechanik (z.B. mobile Röntgensysteme, Röntgen-Computertomographen, Magnetresonanztomographen etc.) eingesetzt. Die Halte- und/oder Führungssysteme sind zur Positionierung der medizinischen Geräte ausgebildet. Die Positionierung der medizinischen Geräte ist dabei durch das Halte- und/oder Führungssystem begrenzt bzw. eingeschränkt. Bildgebende Systeme mit starrer geometrischer Anordnung sind nicht frei bzw. entsprechend den Bedürfnissen des Patienten positionierbar. Medizinische Geräte können beispielsweise Röntgenröhren, Röntgendetektoren, Ultraschallköpfe, Endoskope etc. sein.

Mobil einsetzbare medizinische Geräte werden gegenwärtig von Hand zum Einsatzort transportiert und in der Regel auch händisch für die jeweilige Anwendung positioniert und justiert. Für die bildgestützte Therapie werden bislang verschiedene Systeme (Bildgebung plus Aktorik und ggf. weitere Geräte) aufwändig und zeitintensiv manuell registriert.

Die Erfindung hat zur Aufgabe, Mittel bereitzustellen, die eine räumlich flexible und automatisierte Positionierung von medizinischen Geräten ermöglichen. Des Weiteren hat die Erfindung zur Aufgabe, eine automatisierte statische und dynamische Bildgebung zu ermöglichen und autonome Assistenzfunktionen bereitzustellen.

Diese Aufgabe wird gelöst durch eine mobile Plattform gemäß Anspruch 1. Die Aufgabe wird auch gelöst durch ein System umfassend eine Mehrzahl an mobilen Plattformen gemäß dem nebengeordneten Anspruch. Bevorzugte und/oder alternative, vorteilhafte Ausgestaltungsvarianten sind Gegenstand der abhängigen Ansprüche.

In einem ersten Aspekt betrifft die Erfindung eine mobile Plattform, welche ein Fahrwerk, ein Sensormodul und mindestens eine Haltevorrichtung umfasst. Die mindestens eine Haltevorrichtung ist dabei zum Führen eines ersten medizinischen Gerätes ausgebildet und derart ausgestaltet, das erste medizinische Gerät innerhalb eines Verstellbereiches in mindestens eine Arbeitsposition zu positionieren. Das Sensormodul umfasst mindestens einen Orientierungs-Sensor, welcher ausgebildet ist, wenigstens einen in einer Umgebung der mobilen Plattform angeordneten Referenzpunkt zu erfassen. Das Fahrwerk und/oder die mindestens eine Haltevorrichtung sind ausgebildet, die Arbeitsposition des an der mindestens einen Haltevorrichtung angebrachten ersten medizinischen Gerätes der Bewegung eines zweiten medizinischen Gerätes anzupassen. Das zweite medizinische Gerät wird von einer zweiten mobilen Plattform oder von einer an einer immobilen Plattform angebrachten, zweiten beweglichen Haltevorrichtung geführt, wobei das zweite medizinische Gerät seine Position an die mobile Plattform übermittelt. Das Fahrwerk und/oder die Haltevorrichtung sind ausgebildet, das Anpassen der Arbeitsposition des ersten medizinischen Geräts an die Bewegung des zweiten medizinischen Geräts auszuführen, indem das erste medizinische Gerät abhängig von der übermittelten Position des zweiten medizinischen Geräts positioniert wird.

Das Fahrwerk dient dem Verfahren bzw. Fahren der mobilen Plattform umfassend alle Bestandteile bzw. Komponenten. Insbesondere kann die mobile Plattform mittels des Fahrwerkes verschiedene Positionen einnehmen. Das Fahrwerk umfasst vorteilhafterweise eine Antriebseinheit und eine Transporteinheit. Die Transporteinheit kann mindestens eine Rolle oder mindestens ein Rad oder mindestens eine Walze oder mindestens eine Kette zur Fortbewegung des Fahrwerkes umfassen. Die Antriebseinheit treibt die Transporteinheit an. Die Antriebseinheit ist vorteilhafterweise als Motor, bevorzugt als Elektromotor, ausgebildet. Insbesondere ist der Elektromotor als elektromechanischer Wandler ausgebildet. Mit anderen Worten wandelt der Elektromotor elektrische Energie in mechanische Energie. Alternativ kann der Motor auch ausgebildet sein, thermische, chemische, hydraulische oder pneumatische Energie in mechanische Energie umzuwandeln. Insbesondere kann die mechanische Energie zum Antreiben der Transporteinheit dienen.

Das Sensormodul umfasst wenigstens einen, bevorzugt eine Vielzahl von Sensoren, die der Umgebungserkennung der mobilen Plattform dienen. Der wenigstens eine Sensor ist vorteilhafterweise dazu ausgebildet, wenigstens einen, bevorzugt mehrere Umgebungsparameter zu erfassen. Der Umgebungsparameter kann insbesondere eine Position der mobilen Plattform oder einen Abstand der mobilen Plattform zu anderen Gegenständen, Personen etc. oder eine Position eines Patienten umfassen. Insbesondere können der bzw. die Umgebungsparameter die Bewegung bzw. Position anderer Geräte, Personen etc. umfassen.

Die mindestens eine Haltevorrichtung ist derart ausgebildet, dass sie das erste medizinische Gerät halten und in eine gewünschte Arbeitsposition verstellen bzw. positionieren kann. Insbesondere kann die mindestens eine Haltevorrichtung das erste medizinische Gerät innerhalb eines Verstellbereiches positionieren. Der Verstellbereich kann einen Bereich von 0cm bis 300cm in der Höhe umfassen. Dabei umfasst dieser Bereich vorteilhafterweise einen Bereich zwischen 50cm und 250cm. In Ausführungen umfasst der Verstellbereich die Größe eines beliebigen Patienten. Der Verstellbereich ist in Ausführungen abhängig von der Wahl des ersten medizinischen Gerätes. Das erste medizinische Gerät ist vorteilhafterweise zur Durchführung einer medizinischen Untersuchung oder für eine medizinischen Eingriff oder zur Assistenz bei einem medizinischen Eingriff ausgebildet. Der Verstellbereich wird durch die Ausgestaltung der mindestens einen Haltevorrichtung eingeschränkt. Der Verstellbereich kann abhängig von der Ausgestaltung der Haltevorrichtung in Ausführungsbeispielen einen dreidimensionalen Bereich umfassen, in welchem das erste medizinische Gerät mittels der mindestens einen Haltevorrichtung positionierbar ist. Das Positionieren des ersten medizinischen Gerätes in mindestens eine Arbeitsposition kann dabei in Ausführungen ein Drehen, Kippen oder eine Höhenverstellung des ersten medizinischen Gerätes mit dem Ziel, das erste medizinische Gerät in die mindestens eine Arbeitsposition zu positionieren, umfassen. Die mindestens eine Arbeitsposition wird durch eine Aufgabe des ersten medizinischen Gerätes definiert. In der mindestens einen Arbeitsposition kann das erste medizinische Gerät seine Aufgabe ausführen. Die Aufgabe des medizinischen Gerätes ist beispielsweise die Durchführung einer medizinischen Untersuchung oder eines medizinischen Eingriffs oder einer Assistenzfunktion. Die medizinische Untersuchung kann eine bildgebende Untersuchung eines Patienten, beispielsweise eine Röntgenaufnahme, eine Ultraschallaufnahme, eine Tomographie etc. oder eine Kombination derartiger Bildgebungsverfahren sein. Der medizinische Eingriff kann beispielsweise eine Endoskopie, ein chirurgischer Eingriff, ein minimalinvasiver Eingriff etc. sein. Die Assistenzfunktion kann der Transport von Materialien zu einer medizinischen Untersuchung etc. sein.

Insbesondere kann die mindestens eine Haltevorrichtung derart ausgebildet sein, dass sie das erste medizinische Gerät auf einer vorgegebenen Trajektorie bewegt bzw. führt. In Ausführungen kann die Trajektorie durch einen Bediener vorgegeben werden. Alternativ kann die Trajektorie als Standard-Trajektorie für eine bestimmte Untersuchung festgelegt sein, beispielsweise für eine Tomographie des Bauchraums eines Patienten. Alternativ kann die Trajektorie aus den verschiedenen Umgebungsparametern, die mit dem Sensormodul erfasst werden, instantan berechnet werden.

Das Führen beschreibt dabei das Bewegen des ersten medizinischen Gerätes mittels der mindestens einen Haltevorrichtung. Dabei kann das Bewegen zum Positionieren des ersten medizinischen Gerätes in der mindestens einen Arbeitsposition dienen. Alternativ kann das Bewegen dem Abfahren einer Trajektorie, welche aus einer Mehrzahl an Arbeitspositionen besteht, dienen.

In Ausgestaltungen ist das erste medizinische Gerät aus der folgenden Gruppe medizinischer Geräte: Röntgendetektor, Röntgenröhre, Ultraschallkopf, Endoskop, Arbeitsleuchte, medizinisches Instrument oder medizinisches Material.

Der Röntgendetektor bzw. Detektor ist in einer bevorzugten Ausführungsvariante ein Flachbilddetektor bzw. Röntgenflachbilddetektor. Dabei kann es sich um einen Halbleiter- oder einen Szintillationsdetektor handeln. In einer bevorzugten Ausführungsform ist der Röntgendetektor ein digitaler Röntgendetektor.

Die Röntgenröhre ist vorteilhaferweise eine Drehanoden-Röntgenröhre. Alternativ kann die Röntgenröhre auch eine Transmissionsanoden-Röntgenröhre sein. Die Röntgenröhre umfasst ein Austrittsfenster, aus der die Röntgenstrahlung vorteilhafterweise als Kegelstrahl austritt.

Der Ultraschallkopf kann vorteilhafterweise als Sektor-Scanner, Linear-Scanner oder Konvex-Scanner ausgebildet sein. Der Ultraschallkopf ist zum Aussenden und Empfangen von Ultraschallwellen mit einem Piezokristall ausgebildet.

Das Endoskop ist zum Durchführen minimalinvasiver Eingriffe oder minimalinvasiver Untersuchungen ausgebildet. Das Endoskop kann starr oder flexibel ausgebildet sein. Das Endoskop kann ausgebildet sein, verschiedene medizinische Instrumente, wie Katheter, Ablationsinstrumente, Greifer, Kameras, etc. in einen Patienten einzuführen.

Die Arbeitsleuchte ist zum Ausleuchten eines medizinischen Operationsbereiches oder Untersuchungsbereiches ausgebildet. Die Arbeitsleuchte umfasst ein Leuchtmittel, welches sichtbares Licht aussendet. Das Licht kann vorteilhafterweise diffus oder auf einen kleinen Bereich gerichtet ausgesendet werden.

Insbesondere kann das medizinische Instrument eine Spritze, ein Katheter, ein Ablationsinstrument, Operationsbesteck, eine Kamera etc. sein. Insbesondere kann das medizinische Instrument alle weiteren Instrumente, die in einem Krankenhaus oder in einer Arztpraxis verwendet und bewegt werden müssen, sein. Insbesondere kann das medizinische Material ein Behältnis mit Ultraschallgel, Gaze oder Tupfer sein. Insbesondere kann das medizinische Material alle weiteren Verbrauchsmaterialien, die in einem Krankenhaus oder einer Arztpraxis verwendet werden, sein.

In Ausführungen kann das erste medizinische Gerät jegliches Gerät, welches für eine Diagnose oder die medizinische Untersuchung oder den medizinischen Eingriff oder für eine Assistenz bei dem medizinischen Eingriff bei einem Patienten benötigt wird, sein.

In Ausgestaltungen umfasst die mindestens eine Haltevorrichtung eine Hubvorrichtung, eine Drehvorrichtung, einen robotischen Aktuator und/oder eine Manipulationsvorrichtung.

Insbesondere kann die mobile Plattform ein Gehäuse umfassen, an welchem die Haltevorrichtung befestigt bzw. angeordnet sein kann. Die Bezeichnungen am Gehäuse befestigt und am Fahrwerk befestigt werden im Folgenden synonym verwendet. Das erste medizinische Gerät kann an der Hubvorrichtung oder an der Drehvorrichtung oder an dem robotischen Aktuator oder an der Manipulationsvorrichtung befestigt bzw. angeordnet sein. Insbesondere kann das erste medizinische Gerät von der Hubvorrichtung oder der Drehvorrichtung oder dem robotischen Aktuator oder der Manipulationsvorrichtung geführt werden. Die Bestandteile bzw. Komponenten der mindestens einen Haltevorrichtung können beliebig kombiniert sein. Für eine Kombination werden die Bestandteile miteinander gekoppelt, das heißt, die Bestandteile werden aneinander befestigt. Insbesondere kann beispielsweise wenigstens eine dieser Befestigungen lösbar ausgebildet sein. Insbesondere kann eine lösbare Befestigung durch wenigstens eine Schraube oder einen Clip oder eine Nut oder einen Schnappverschluss, etc. ausgebildet sein. Die Bezeichnungen befestigt und gekoppelt werden synonym verwendet. Bestandteile bzw. Komponenten der Haltevorrichtung können die Hubvorrichtung, die Drehvorrichtung, der robotische Aktuator und oder die Manipulationsvorrichtung sein.

Die Hubvorrichtung ist zum Ausführen linearer Verstellbewegungen ausgestaltet. Bevorzugt dient sie der Höhenverstellung des ersten medizinischen Gerätes in vertikaler Richtung. Die Hubvorrichtung deckt die Arbeitspositionen des ersten medizinischen Gerätes in der Höhe ab. Vorteilhafterweise umfasst die Hubvorrichtung eine Höhe von 0cm bis 300cm. Die umfasste Höhe kann von dem ersten medizinischen Gerät bzw. von der Aufgabe des ersten medizinischen Gerätes abhängen, kann aber auch universell für beliebige medizinische Geräte bereitgestellt werden, sodass die mobile Plattform besonders flexibel eingesetzt werden kann. In Ausführungen kann die Hubvorrichtung dabei teleskopartig ausgebildet sein und für das Einstellen kleinerer Höhen mittels in der Höhe immer schmalerer werdender Teleskop-Abschnitte ineinander verfahrbar sein. In alternativen Ausführungen entspricht die Hubvorrichtung einer Schiene, die der Höhenverstellung dient. Das erste medizinische Gerät kann direkt an der Hubvorrichtung befestigt sein. Alternativ kann an der Hubvorrichtung auch ein weiterer möglicher Bestandteil der Haltevorrichtung befestigt sein.

Die Drehvorrichtung kann an der Hubvorrichtung befestigt sein. Insbesondere kann das erste medizinische Gerät direkt an der Drehvorrichtung befestigt sein. Die Drehvorrichtung kann derart ausgebildet sein, dass ein an der Drehvorrichtung befestigtes erste medizinisches Gerät um die Hubvorrichtung herumgedreht werden kann.

Die Drehvorrichtung kann in Ausführungen als Ring ausgebildet sein, der entlang der Hubvorrichtung in der Höhe verstellt werden kann. Insbesondere kann dann die Drehvorrichtung entlang der Hubvorrichtung bis auf eine minimale Höhe, welche durch die Höhe des Gehäuses vorgegeben wird, verstellt werden. Tiefere Positionen, das heißt Positionen des ersten medizinischen Gerätes näher am Boden, werden realisiert, indem das erste medizinische Gerät beispielsweise an einem robotischen Aktuator befestigt ist, welcher das erste medizinische Gerät niedriger als die minimale Höhe positionieren kann. Insbesondere ist ein Drehen des an der Drehvorrichtung befestigten ersten medizinischen Gerätes in einem Winkelbereich zwischen 0° und 360° möglich. In Ausführungen kann die Drehvorrichtung das erste medizinische Gerät in kleineren Winkelbereichen drehen. Vorteilhafterweise deckt der Winkelbereich wenigstens 90° auf der Seite der Hubvorrichtung ab, die gegenüberliegend zu dem Gehäuse der mobilen Plattform angeordnet ist. Insbesondere kann die Drehvorrichtung das erste medizinische Gerät in Richtung und/oder entgegen des Uhrzeigersinnes drehen.

In alternativen Ausführungen kann die Drehvorrichtung als Teil-Ring oder als Drehgelenk ausgebildet sein. Insbesondere kann dann die Drehvorrichtung entlang der gesamten Höhe der Hubvorrichtung verstellt werden. Insbesondere kann die Drehvorrichtung das erste medizinische Gerät beispielsweise in einem Winkelbereich zwischen 0° und 180° drehen. Analog zu der Ausführung als Ring kann die Drehvorrichtung in Ausführungen das erste medizinische Gerät in kleineren Winkelbereichen drehen. Analog kann die Drehung in Richtung und/oder entgegengesetzt des Uhrzeigersinnes ausgeführt werden.

Der robotische Aktuator ist derart ausgestaltet, dass er eine dreidimensionale Bewegung des ersten medizinischen Gerätes ermöglicht und somit eine Positionierung des ersten medizinischen Gerätes horizontal entfernt von dem Gehäuse und von dem Fahrwerk ermöglicht. Das Fahrwerk und das Gehäuse sind vorteilhafterweise fest verbunden. Damit ist die Bezeichnung "vom Gehäuse beabstandet" synonym zu der Bezeichnung "von dem Fahrwerk beabstandet". Das erste medizinische Gerät kann in Ausführungen direkt an dem robotischen Aktuator befestigt sein.

In Ausführungen umfasst der robotische Aktuator wenigstens ein Dreh- und/oder Scharniergelenk. Insbesondere kann der robotische Aktuator den horizontalen Abstand des ersten medizinischen Gerätes von dem Gehäuse durch eine Variation eines Winkels des Dreh- und/oder Scharniergelenkes variieren. Der Winkel des Dreh- und/oder Scharniergelenkes kann vorteilhafterweise einen maximalen Winkelbereich zwischen 0° und 180° umfassen. In Ausführungen kann der Winkelbereich einen Teilbereich des maximalen Winkelbereiches umfassen. Der Abstand des ersten medizinischen Gerätes von dem Gehäuse ist maximal, wenn der robotische Aktuator maximal ausgefahren ist. Insbesondere ist im maximal ausgefahrenen Zustand der Winkel des Dreh- und/oder Scharniergelenkes der maximale Winkel des Winkelbereiches.

Der robotische Aktuator kann in Ausführungen das medizinische Gerät um beliebige Achsen drehen bzw. kippen. Der Drehwinkel wird typischerweise von der Ausführung des robotischen Aktuators und des ersten medizinischen Gerätes begrenzt. Beispielsweise können die Drehwinkel einen Bereich zwischen 0° und 120° umfassen. Insbesondere kann der robotische Aktuator mittels einer Drehung die Ausrichtung des ersten medizinischen Gerätes verstellen.

In Ausführungen kann der robotische Aktuator das erste medizinische Gerät in verschiedenen horizontalen Abständen zum Gehäuse positionieren. In Ausführungen kann der robotische Aktuator ein erstes medizinisches Gerät in der Höhe verstellen bzw. positionieren. Insbesondere ist die Höhenverstellbarkeit abhängig von dem Abstand des ersten medizinischen Gerätes zum Gehäuse. Je größer der Abstand des ersten medizinischen Gerätes vom Gehäuse, desto geringer ist der Verstellbereich in der Höhe, der mit dem robotischen Aktuator realisiert werden kann. Insbesondere kann das erste medizinische Gerät bei gleichbleibendem horizontalem Abstand zum Gehäuse nicht in der Höhe verstellt werden, wenn der robotische Aktuator maximal ausgefahren ist.

Der robotische Aktuator kann in Ausführungen an der Hubvorrichtung befestigt sein, sodass er in der Höhe verstellt werden kann. In dieser Ausführung kann das erste medizinische Gerät in der Höhe mittels der Hubvorrichtung grob positioniert werden. Eine Feinjustierung der Höhe, der Ausrichtung und/oder des Abstands zum Gehäuse ist mittels des robotischen Aktuators möglich.

Der robotische Aktuator kann in Ausführungen direkt am Gehäuse befestigt sein. In diesen Ausführungen ist die Höhenverstellbarkeit des ersten medizinischen Gerätes auf die Reichweite des robotischen Aktuators in dem entsprechend eingestellten horizontalen Abstand zum Gehäuse beschränkt.

In Ausführungen kann der robotische Aktuator an der Drehvorrichtung befestigt sein.

Die Manipulationsvorrichtung umfasst einen robotischen Aktuator und eine Greifvorrichtung. Das erste medizinische Gerät kann mittels der Greifvorrichtung von der Manipulationsvorrichtung aufgenommen bzw. gegriffen und abgelegt werden. Die Greifvorrichtung kann dabei in Ausführungen eine automatische Greifvorrichtung sein. Alternativ kann die Greifvorrichtung auch eine manuelle Klemme oder eine Spannvorrichtung oder Haken zum Einhaken an dem ersten medizinischen Gerät oder Magnete etc. umfassen. Die Manipulationsvorrichtung kann analog zu dem robotischen Aktuator in Ausführungen entweder direkt am Gehäuse, an der Hubvorrichtung oder an der Drehvorrichtung befestigt sein.

Im Folgenden wird der Ausdruck "das erste medizinische Gerät ist an der mobilen Plattform befestigt" synonym zu der Bedeutung, dass das erste medizinische Gerät an der mindestens einen beliebig ausgebildeten Haltevorrichtung befestigt ist, verwendet.

Die Erfinder haben erkannt, dass mit dieser Erfindung eine mobile Plattform als Basis für ein weites Spektrum von medizinischen Applikationen bereitgestellt werden kann. Durch jeweils wenigstens eine vertikale Hubvorrichtung, Drehvorrichtung, robotischen Aktuator und/oder Manipulationsvorrichtung kann die Plattform so ausgestaltet werden, dass sie uneingeschränkt alle denkbaren Arbeitsräume vom Fußboden bis über die Körpergröße eines Patienten abdecken kann. Damit gibt es keinerlei Limitierungen des möglichen Arbeitsraums innerhalb der Begrenzungen durch das umgebende Gebäude. Somit sind beispielsweise abhängig von der Ausbildung des ersten medizinischen Gerätes verschiedene medizinische Bildaufnahmen wie Röntgen, Ultraschall etc. möglich. Insbesondere können medizinische Eingriffe und/oder medizinische Untersuchungen von der mobilen Plattform ausgeführt werden.

Die Erfinder haben weiterhin erkannt, dass die mobile Plattform unter anderem Assistenzfunktionen für den medizinischen Workflow und Transportaufgaben übernehmen kann. Beispielsweise kann die mobile Plattform mit dem Greifer der Manipulationsvorrichtung das erste medizinische Gerät aufnehmen und an einen Ort fahren, an dem das erste medizinische Gerät benötigt wird.

Das Sensormodul umfasst mindestens einen Orientierungs-Sensor, welcher ausgebildet ist, wenigstens einen in einer Umgebung der mobilen Plattform angeordneten Referenzpunkt zu erfassen.

Durch das Erfassen des Referenzpunktes bzw. Referenzmarkers ist die mobile Plattform in einer Umgebung koordiniert bzw. orientiert. Mit anderen Worten bedeutet das, dass die mobile Plattform mittels des Referenzpunktes bestimmen kann, wo sie sich in ihrer Umgebung befindet. Mit anderen Worten kann die mobile Plattform mittels des Referenzpunktes ihre Position bestimmen. Insbesondere umfasst der wenigstens eine Umgebungsparameter den wenigstens einen Referenzpunkt. Der wenigstens eine Referenzpunkt kann als Fixpunkt innerhalb eines Koordinatensystems ausgebildet sein, in welchem die mobile Plattform orientiert ist. Das Koordinatensystem kann die Umgebung der mobilen Plattform beschreiben. Die Umgebung kann dabei beispielsweise ein Operationssaal, eine Radiologie, ein Behandlungsraum oder eine ganze Klinik sein. Insbesondere ermöglicht die Orientierung der mobilen Plattform in ihrer Umgebung das Anfahren definierter Punkte bzw. Orte in der Umgebung. Definierte Punkte bzw. Orte können innerhalb eines Raumes, an einer Patientenliege, eine Parkposition der mobilen Plattform etc. sein.

Ein Referenzpunkt kann in Ausführungen optisch oder elektromagnetisch ausgebildet sein. Alternativ kann es sich bei dem Referenzpunkt um wenigstens ein Schallsignal handeln.

In alternativen Ausführungen bestimmt der mindestens eine Orientierungs-Sensor die Position der mobilen Plattform in der Umgebung mittels Odometrie oder durch Tracking und Vermessung der Umgebung. Das Bestimmen kann optisch, inertial, akustisch oder funktechnisch durchgeführt werden.

Die Erfinder haben erkannt, dass durch die Koordinierung bzw. Orientierung der mobilen Plattform in der Umgebung der mobilen Plattform ein selbsttätiges Bewegen der mobilen Plattform zu einem vorgegebenen Punkt bzw. Ort in der Umgebung möglich ist. Mit anderen Worten ist es möglich, dass die mobile Plattform voll-autonom verfahrbar ist. Alternativ oder zusätzlich ist es möglich, dass die mobile Plattform spezifische Trajektorien abfährt. Trajektorien können Bewegungsbahnen bzw. Wege in der Umgebung sein. Vorteilhafterweise können Trajektorien eine Aneinanderreihung von Punkten bzw. Positionen in der Umgebung sein, die die mobile Plattform nacheinander abfährt. Die Trajektorien können von der mobilen Plattform mittels des Fahrwerks abgefahren werden. Vorteilhafterweise können die Punkte der Trajektorien Arbeitspositionen des ersten medizinischen Gerätes entsprechen. Insbesondere können die Trajektorien von dem ersten medizinischen Gerät mittels der Haltevorrichtung abgefahren werden. Insbesondere können die Trajektorien von dem ersten medizinischen Gerät durch ein Führen der Haltevorrichtung und ein gleichzeitiges Fahren des Fahrwerkes abgefahren werden.

Wird im Folgenden von dem Abfahren einer Trajektorie gesprochen, kann das das Abfahren mit dem Fahrwerk und/oder mit der mindestens einen Haltevorrichtung betreffen. Insbesondere kann es das Abfahren mit der mobilen Plattform als Ganzes und das Abfahren mit dem ersten medizinischen Gerät betreffen.

Außerdem haben die Erfinder erkannt, dass für eine Orientierung der mobilen Plattform durch wenigstens einen Referenzpunkt keine Umbaumaßnahmen in der Umgebung der mobilen Plattform notwendig sind. Der Referenzpunkt kann an die Bauweise angepasst werden und die mobile Plattform kann sich in der Umgebung anhand des Referenzpunktes orientieren.

In Ausgestaltungen umfasst das Sensormodul mindestens einen Kollisions-Sensor, welcher ausgebildet ist, Objekte in der Umgebung der mobilen Plattform zu erkennen.

Durch die Erkennung von Objekten in der Umgebung der mobilen Plattform kann ein Zusammenstoßen der mobilen Plattform beim Fahren der mobilen Plattform mittels des Fahrwerkes mit einem dieser Objekte verhindert werden. Der Kollisions-Sensor kann beispielsweise optisch, akustisch, elektromagnetisch, taktil, kapazitiv oder aerodynamisch ausgebildet sein. Objekte können beispielsweise Wände, Tische, Stühle, Geräte, medizinische Geräte, andere mobile Plattformen, Menschen usw. sein. Wird ein Objekt erkannt, bleibt die mobile Plattform entweder stehen oder ändert alternativ ihre Fahrtrichtung, um das Objekt zu umfahren. Insbesondere umfasst der wenigstens eine Umgebungsparameter das mit dem Kollisions-Sensor erkannte Objekt.

In bevorzugten Ausführungen kann der Kollisions-Sensor zusätzliche einen Abstands-Sensor umfassen. Vorteilhafterweise ist der Abstands-Sensor in den Kollisions-Sensor integriert. Mit anderen Worten bilden der Abstands-Sensor und der Kollisions-Sensor einen kombinierten Sensor. Der Abstands-Sensor kann auf denselben Techniken (optisch, akustisch, elektromagnetisch, taktil, kapazitiv oder aerodynamisch) wie der Kollisions-Sensor basieren. Mit dem Abstands-Sensor ist es möglich die mobile Plattform gezielt, bis auf einen definierten Abstand an ein Objekt heranzufahren und die mobile Plattform zu stoppen, bevor sie mit dem Objekt kollidiert. Insbesondere kann der wenigstens eine Umgebungsparameter den Abstand der mobilen Plattform zu einem Objekt umfassen.

In besonders bevorzugten Ausführungen umfasst die mobile Plattform Kollisions- und in Ausführungen Abstands-Sensoren in alle möglichen Fahrtrichtungen des Fahrwerkes. Vorteilhafterweise kann derart unabhängig von der Fahrtrichtung der mobilen Plattform eine Kollision bei jeder Positionsänderung/jedem Verfahren der mobilen Plattform verhindert werden.

In besonders bevorzugten Ausführungsformen ist mindestens ein Kollisions-Sensor und in Ausführungen mindestens ein Abstands-Sensor an der mindestens einen Haltevorrichtung angeordnet. Insbesondere ist es somit möglich, ein Kollidieren des ersten medizinischen Gerätes mit einem Objekt zu verhindern, wenn das erste medizinische Gerät mit der mindestens einen Haltevorrichtung positioniert wird. Insbesondere kann somit das erste medizinische Gerät bis auf einen definierten Abstand an einen Patienten heranfahren bzw. in einem definierten Abstand zu einem Patienten positioniert werden.

Die Erfinder haben erkannt, dass der Kollisions-Sensor dem Schutz von Geräten und Menschen dient. Außerdem haben die Erfinder erkannt, dass mittels eines in den Kollisions-Sensor integrierten Abstands-Sensors ein gezieltes Fahren der mobilen Plattform und/oder des ersten medizinischen Gerätes an einen Punkt in einem definierten Abstand zu einem Objekt möglich ist. Beispielsweise kann das erste medizinische Gerät ein Röntgendetektor sein, welcher auf diese Weise bis auf einen festgelegten Abstand an einen Patienten herangefahren werden kann, ohne mit dem Patienten zu kollidieren. Insbesondere kann die mobile Plattform mit Hilfe des Kollisions-Sensors in einer Parklücke zwischen anderen Objekten einparken, ohne mit diesen zu kollidieren.

Außerdem haben die Erfinder erkannt, dass mit dem Kollisions-Sensor und/oder dem Orientierungs-Sensor ein teil-autonomes Verfahren der mobilen Plattform möglich ist. Teil-autonom bedeutet, dass das Verfahren von einem Bediener begleitet wird, die mobile Plattform allerdings selbsttätig beispielsweise mit dem Orientierungs-Sensor die Fahrspur hält und/oder mit dem Kollisions-Sensor Kollisionen vermeidet. Außerdem kann die mobile Plattform selbsttätig in ihre Parkposition verfahren. Alternativ ist es mit dem Kollisions-Sensor und/oder dem Orientierungs-Sensor möglich, dass die mobile Plattform voll-autonom verfahrbar ist. Somit kann die mobile Plattform in einer beliebigen Ausprägung zwischen teil-autonom bis voll-autonom verfahrbar sein.

In Ausgestaltungen umfasst das Sensormodul mindestens einen Patientenerkennungs-Sensor.

Der Patientenerkennungs-Sensor umfasst vorzugsweise eine Kamera, mit welcher ein Patient bildlich erfasst werden kann. In Ausführungen wird in das Bild des Patienten ein Skelett gefittet, um Körperregionen bzw. Landmarken in der Physiologie des Patienten zuordnen zu können. Mit anderen Worten kann der Patientenerkennungs-Sensor Körperregionen eines Patienten erkennen. Mit der Körperregionserkennung kann die mobile Plattform verschieden Körperregionen des Patienten erkennen und räumlich zuordnen. Insbesondere kann der wenigstens eine Umgebungsparameter einen beispielsweise bildlich erfassten Patienten umfassen. In Ausführungsbeispielen kann sich die mobile Plattform in einem ersten Schritt wenigstens einem Referenzpunkt an einem Patiententisch, auf dem der Patient liegt, orientieren. Anhand dieses Referenzpunktes kann die mobile Plattform zunächst die Patientenliege abfahren und währenddessen mit dem Patientenerkennungs-Sensor den gesamten Patienten erfassen. Die Daten aus dieser Erfassung können wie oben beschrieben durch einen Fit eines Skelettes weiterverarbeitet werden. In einer vereinfachten Ausführung erkennt der Patientenerkennungs-Sensor nur den Kopf und die Füße des Patienten und so seine Ausrichtung in der Umgebung. In Ausführungen kann der Patientenerkennungs-Sensor auch einen stehenden oder sitzenden Patienten erkennen.

Die Erfinder haben erkannt, dass der Patientenerkennungs-Sensor die autonome Positionierung der mobilen Plattform relativ zu der Anatomie bzw. Physiologie eines Patienten ermöglicht. Beispielsweise kann sich die mobile Plattform für eine Ultraschalluntersuchung des Bauches eines Patienten an eine geeignete Stelle positionieren, welche mit dem Patentenerkennungs-Sensor und der Körperregionserkennung von der mobilen Plattform bzw. dem Sensormodul der mobilen Plattform bestimmt wurde. Somit ist eine manuelle Positionierung der mobilen Plattform durch einen Arzt nicht notwendig. Dieses Beispiel kann auf weitere Untersuchungen und Assistenzfunktionen übertragen werden. Insbesondere ist es mittels des Patientenerkennungs-Sensors möglich, dass die mobile Plattform eigenständige Untersuchungen an einem Patienten durchführen kann. Insbesondere haben die Erfinder erkannt, dass die mobile Plattform mit dem Patientenerkennungs-Sensor anhand der Physiologie eines Patienten in der Umgebung des Patienten orientiert sein kann.

Das Fahrwerk und/oder die mindestens eine Haltevorrichtung sind dazu ausgebildet, die Arbeitsposition des an der mindestens einen Haltevorrichtung angebrachten ersten medizinischen Gerätes der Bewegung eines zweiten medizinischen Gerätes anzupassen.

Mit anderen Worten kann die Arbeitsposition bzw. Position des ersten medizinischen Gerätes der Position des zweiten medizinischen Gerätes angepasst sein. Insbesondere ist eine koordinierte Bewegung von zwei medizinischen Geräten zueinander möglich. Die koordinierte Bewegung kann in Ausführungen nachgeführt sein. Insbesondere kann die Bewegung des ersten medizinischen Gerätes der Bewegung des zweiten medizinischen Gerätes nachgeführt sein. Mit anderen Worten kann die Position des ersten medizinischen Gerätes der Position des zweiten medizinischen Gerätes nachgeführt sein. Das bedeutet mit anderen Worten, dass die mobile Plattform und/oder das erste medizinische Gerät die Bewegung und/oder die Position des zweiten medizinischen Gerätes erfasst. Das Erfassen der Bewegung bzw. Position des zweiten medizinischen Gerätes erfolgt dadurch, dass das zweite medizinische Gerät seine Position an die mobile Plattform übermittelt

Über die Orientierung im Raum bzw. der Umgebung der mobilen Plattform kennt die mobile Plattform somit die Position des zweiten medizinischen Gerätes relativ zu der Position des ersten medizinischen Gerätes. Somit kann die mobile Plattform das erste medizinische Gerät entsprechend bzw. abhängig von der Position des zweiten medizinischen Gerätes positionieren bzw. nachführen. Mit anderen Worten kann das erste medizinische Gerät somit der Bewegung bzw. Position des zweiten medizinischen Gerätes folgen. Das Nachführen der Position bzw. der Bewegung des ersten medizinischen Gerätes kann mittels der Haltevorrichtung und/oder mittels des Fahrwerkes der mobilen Plattform realisiert werden.

Das zweite medizinische Gerät kann auf verschiedene Weise bewegt bzw. positioniert bzw. geführt werden. In Ausführungen kann das zweite medizinische Gerät von einer zweiten mobilen Plattform umfasst werden.

Alternativ kann das zweite medizinische Gerät von einer immobilen Plattform bewegt werden. Die immobile Plattform ist fest in der Umgebung positioniert. Die immobile Plattform kann mittels einer Haltevorrichtung umfassend eine Hubvorrichtung, eine Drehvorrichtung, einen robotischen Aktuator und/oder eine Manipulationsvorrichtung ein zweites medizinisches Gerät führen. Die Haltevorrichtung der immobilen Plattform kann an der Decke, am Boden oder an der Wand eines Raumes angeordnet sein. Weitere Ausführungen zu der immobilen Plattform folgen in der weiteren Beschreibung.

In Ausführungen kann die Bewegung bzw. Position des zweiten medizinischen Gerätes der Bewegung bzw. Position des ersten medizinischen Gerätes nachgeführt sein.

In Ausführungen können das erste und das zweite medizinische Gerät jeweils eine festgelegte Trajektorie abfahren. Insbesondere kann diese Trajektorie von einem Nutzer im Vorhinein festgelegt sein. Alternativ kann die Trajektorie automatisch vorab festgelegt sein, bspw. in Abhängigkeit einer geplanten Untersuchung. Alternativ kann die Trajektorie mittels wenigstens eines Sensors des Sensormoduls der mobilen Plattform instantan aus wenigstens einem Umgebungsparameter bestimmt werden und automatisiert so festgelegt werden, dass beispielsweise definierte Körperbereiche eines Patienten mittels der Trajektorie abgefahren werden.

Die Erfinder haben erkannt, dass durch ein Nachführen eines ersten medizinischen Gerätes zu einem zweiten medizinischen Gerät ein automatisierter Untersuchungsablauf an einem Patienten ermöglicht wird.

In einem Ausführungsbeispiel kann das erste medizinische Gerät als Röntgendetektor und das zweite medizinische Gerät als Röntgenröhre ausgebildet sein. Die Röntgenröhre kann an einer immobilen Plattform befestigt sein. Insbesondere kann die Position bzw. Bewegung des Röntgendetektors der Position bzw. Bewegung der Röntgenröhre nachgeführt sein. Alternativ kann die Position bzw. Bewegung der Röntgenröhre der Position bzw. Bewegung des Röntgendetektors nachgeführt sein. Insbesondere kann somit eine Mehrzahl an Röntgenaufnahmen entlang einer Trajektorie automatisiert aufgenommen werden, analog beispielsweise zu einer C-Bogen-Aufnahme oder einer Tomographie.

In einem alternativen, nicht beanspruchtem Ausführungsbeispiel ist das erste medizinische Gerät als Arbeitsleuchte ausgebildet. Das zweite medizinische Gerät kann von der Hand eines Chirurgen geführt werden. Das zweite medizinische Gerät kann beispielsweise ein Skalpell sein. Vorteilhafterweise kann die Position bzw. Bewegung der Arbeitsleuchte jeder Bewegung der Hand des Chirurgen bzw. des Skalpells nachgeführt sein. Dies kann den Arbeitsablauf während einer Operation erleichtern, da kein manuelles Nachstellen der Arbeitsleuchte notwendig ist.

In Ausgestaltungen der Erfindung umfasst die mobile Plattform mindestens zwei Haltevorrichtungen. Die Haltevorrichtungen sind dabei jeweils zum Führen eines ersten und eines dritten medizinischen Gerätes ausgebildet. Die Haltevorrichtungen sind weiterhin dazu ausgestaltet, jeweils das erste und das dritte medizinische Gerät innerhalb jeweils eines Verstellbereiches in jeweils mindestens eine Arbeitsposition zu positionieren.

Insbesondere kann das dritte medizinische Gerät als das zweite medizinische Gerät ausgebildet sein, welches auf der mobilen Plattform angeordnet ist. In dieser Ausführung wird das erste medizinische Gerät auf der Plattform mittels der entsprechenden Haltevorrichtung der Bewegung des dritten medizinischen Gerätes nachgeführt. Insbesondere kann das dritte medizinische Gerät eine vordefinierte bzw. vorgeplante Trajektorie abfahren. Mit anderen Worten kann das dritte medizinische Gerät von der entsprechenden Haltevorrichtung entlang der Trajektorie geführt werden. Insbesondere kann die Trajektorie durch einen Anwender vorgegeben werden. Alternativ kann die mobile Plattform anhand der Patientenerkennung oder der Orientierung im Raum die Trajektorie des dritten medizinischen Geräts instantan berechnen. Alternativ kann die Trajektorie instantan aus wenigstens einem Referenzpunkt, welcher beispielsweise in der Umgebung, am Patienten, am Patiententisch usw. angeordnet ist, berechnet werden. Alternativ kann die Trajektorie aus Bilddaten instantan durch die mobile Plattform berechnet werden. Die Bilddaten werden vorzugsweise mit einem der beiden medizinischen Geräte oder einem weiteren medizinischen Gerät auf einer anderen mobilen oder immobilen Plattform erfasst.

Alternativ können das zweite und das dritte medizinische Gerät verschieden sein. Insbesondere kann das zweite medizinische Gerät an einer weiteren mobilen Plattform oder an einer immobilen Plattform befestigt sein. Alternativ kann das zweite medizinische Gerät manuell bewegt werden. Das erste und das dritte medizinische Gerät können beide der Bewegung des zweiten medizinischen Gerätes nachgeführt werden.

Vorteilhaftweise kann die Haltevorrichtung des ersten und des dritten medizinischen Gerätes teilweise gemeinsame Komponenten umfassen. Bei einer teilweise gemeinsamen Haltevorrichtung ist mindestens eine der Komponenten bzw. Bestandteile der Haltevorrichtungen des ersten und des zweiten medizinischen Gerätes zum Führen beider medizinischer Geräte gleichzeitig ausgebildet.

In Ausführungsbeispielen können das erste und das dritte medizinische Gerät an jeweils einem robotischen Aktuator befestigt sein. Insbesondere können beide robotischen Aktuatoren an einer gemeinsamen Hubvorrichtung befestigt sein. Alternative Ausgestaltungen einer teilweise gemeinsamen Haltevorrichtung des ersten und des zweiten medizinischen Gerätes sind denkbar.

In Ausführungsbeispielen kann das erste medizinische Gerät ein Röntgendetektor und das dritte medizinische Gerät eine Röntgenröhre sein. Die Haltevorrichtungen des Röntgendetektors und der Röntgenröhre können derart ausgebildet sein, dass die Röntgenröhre und der Röntgendetektor in einer vorgeplanten Trajektorie um einen Patienten herumfahren können. Auf diese Weise können wie bei einer C-Bogen-Aufnahme, Röntgenaufnahmen aus verschiedenen Perspektiven des Patienten aufgenommen werden.

Die Erfinder haben erkannt, dass das Führen bzw. Bewegen bzw. Positionieren von zwei medizinischen Geräten an einer gemeinsamen mobilen Plattform den Vorteil mit sich bringt, dass die beiden medizinischen Geräte stabil relativ zueinander bewegt werden können, da sie mit der mobilen Plattform einen gemeinsamen Bezugspunkt haben. Dafür ist keine weitere Orientierung im Raum notwendig. Fehler in der räumlichen Orientierung der Plattform haben keinen Einfluss auf die relative Bewegung der beiden medizinischen Geräte, die an einer gemeinsamen mobilen Plattform befestigt sind. Zudem ist der Platzbedarf geringer, wenn zwei medizinische Geräte an einer gemeinsamen mobilen Plattform befestigt sind, als wenn zwei medizinische Geräte jeweils an einer einzelnen mobilen Plattform befestigt sind.

In Ausgestaltungen der Erfindung umfasst die mobile Plattform ein Batteriemodul. Das Batteriemodul dient der Energieversorgung der mobilen Plattform, insbesondere der Antriebseinheit für das Fahrwerk, aber auch der Motorik für die Haltevorrichtung, etc. Vorteilhafterweise schränkt das Batteriemodul die Mobilität der mobilen Plattform nicht ein. Insbesondere kann das Batteriemodul komplett auf dem Fahrwerk der mobilen Plattform angeordnet sein. Insbesondere benötigt das Batteriemodul keine weiteren permanenten Bauteile, die außerhalb der mobilen Plattform bzw. in der Umgebung der mobilen Plattform angeordnet sind, wie beispielsweise Kabel.

Vorteilhafterweise ist das Batteriemodul aufladbar ausgebildet. Insbesondere ist es möglich, das Batteriemodul zu laden, wenn die mobile Plattform nicht benötigt wird. Beispielsweise kann das Batteriemodul nachts laden oder wenn eine andere mobile Plattform die Aufgabe der mobilen Plattform erfüllen kann.

Alternativ dazu kann die mobile Plattform mittels flexibler Kabellösungen, kabelloser Energieübertragung wie Induktion oder alternativer systemimmanenter Speicherlösungen wie Brennstoffzellen mit Energie versorgt werden.

Die Erfinder haben erkannt, dass die Verwendung eines Batteriemoduls ein flexibles Einsetzen der mobilen Plattform ermöglicht. Vorteilhafterweise werden für ein Batteriemodul keine permanenten externen Bauteile wie Kabel benötigt, die die Mobilität der mobilen Plattform einschränken und das Verletzungsrisiko von Patienten und Bedienpersonal durch Stolperfallen erhöhen können.

In Ausgestaltungen der Erfindung umfasst die mobile Plattform einen Kippschutz.

Der Kippschutz dient insbesondere dazu, dass die mobile Plattform auch dann nicht kippt bzw. umkippt, wenn das erste und/oder dritte medizinische Gerät mit der Haltevorrichtung horizontal entfernt vom Schwerpunkt der mobilen Plattform in eine Arbeitsposition positioniert wird.

Der Kippschutz kann insbesondere durch einen tief liegenden Schwerpunkt der mobilen Plattform ausgebildet sein. Ein tief liegender Schwerpunkt ist innerhalb der mobilen Plattform möglichst weit unten angeordnet. Mit anderen Worten ist ein tief liegender Schwerpunkt möglichst nah am Boden am Fahrwerk der mobilen Plattform angeordnet.

Alternativ oder zusätzlich kann der Kippschutz durch eine große Grundfläche ausgebildet sein. Eine große Grundfläche bedeutet, dass das Fahrwerk der mobilen Plattform eine große Fläche umfasst. Die Größe der Grundfläche darf jedoch die Mobilität bzw. Wendigkeit der mobilen Plattform nicht einschränken.

In Ausführungsbeispielen kann das Gewicht der mobilen Plattform als Kippschutz dienen. Dafür muss die mobile Plattform derart schwer ausgebildet sein, dass das Gewicht der mobilen Plattform die Hebelwirkung durch ein an der mobilen Plattform befestigtes erstes und/oder drittes medizinisches Gerät ausgleichen kann.

In Ausführungsbeispielen kann der Kippschutz wenigstens einen Beschleunigungssensor im Sensormodul umfassen, der ein Kippen der mobilen Plattform detektiert und mit einer Ausgleichsbewegung der mindestens einen Haltevorrichtung automatisiert verhindert. Beispielsweise kann der Beschleunigungssensor schräge Bewegungen der mobilen Plattform bzw. Bewegungen, die im normalen Gebrauch der mobilen Plattform nicht auftreten, erkennen. Insbesondere kann der Beschleunigungssensor beim Erkennen einer solchen Bewegung der mobilen Plattform diese Information an die Haltevorrichtung weiterleiten. Insbesondere kann der Bewegungssensor eine Information über die Richtung dieser Bewegung der mobilen Plattform an die Haltevorrichtung weiterleiten. Die Haltevorrichtung kann durch ein Bewegen des ersten medizinischen Gerätes den Schwerpunkt der mobilen Plattform entgegen der Kippbewegung verlagern. Das Verlagern des Schwerpunktes kann ein Kippen der mobilen Plattform verhindern.

Alternativ kann der Kippschutz vorgegebene Bedingungen umfassen, die festlegen, wie weit in horizontaler Richtung entfernt vom Schwerpunkt der mobilen Plattform ein erstes und/oder drittes medizinisches Gerät mit der mindestens einen Haltevorrichtung positioniert werden darf, sodass die mobile Plattform nicht kippt. Dabei kann diese Bedingung vom Gewicht und der Ausgestaltung des ersten und/oder dritten medizinischen Gerätes abhängen.

Insbesondere bestimmt der Verstellbereich, den die mindestens eine Haltevorrichtung umfasst, die Ausbildung des Kippschutzes. Je größer der Bewegungsradius des Verstellbereiches ist, mit anderen Worten, je weiter das erste und/oder dritte medizinische Gerät mit der mindestens einen Haltevorrichtung horizontal vom Schwerpunkt der mobilen Plattform entfernt positioniert werden kann, desto stabiler muss der Kippschutz ausgestaltet sein.

Die Erfinder haben erkannt, dass insbesondere durch einen tiefliegenden Schwerpunkt und/oder eine große Grundfläche der mobilen Plattform das Risiko, dass die mobile Plattform kippt, minimiert werden kann. Vorteilhafterweise ist die Größe der Grundfläche der mobilen Plattform derart beschränkt, dass sie durch eine Tür passt. Insbesondere kann die Grundfläche beispielsweise eine Fläche von 80cm x 80cm umfassen. Das Gewicht einer solchen Plattform ohne das erste medizinische Gerät kann beispielsweise 80kg betragen. Außerdem haben die Erfinder erkannt, dass die Ausgestaltung des Kippschutzes vorteilhafterweise abhängig von dem ersten und/oder dritten medizinischen Gerät, welches an der mobilen Plattform befestigt ist, und von dem Bewegungsradius der Haltevorrichtung ist.

In Ausgestaltungen der Erfindung umfasst die mobile Plattform eine Ablagefläche. Insbesondere kann die Oberseite des Gehäuses der mobilen Plattform als Ablagefläche ausgebildet sein. Vorteilhafterweise sind keine zusätzlichen Bauteile zur Ausgestaltung der Ablagefläche notwendig. Vorteilhafterweise befindest sich die Ablagefläche auf Tischhöhe, sodass sie dem Bedienpersonal und/oder dem Patienten zum Ablegen von Gegenständen dient. Vorteilhafterweise ist die Ablagefläche derart ausgebildet, dass sie einfach desinfiziert werden kann. Vorteilhafterweise ist die Ablagefläche genauso groß, wie die Grundfläche der mobilen Plattform. Somit vergrößert die Ablagefläche den Platzbedarf der mobilen Plattform nicht.

Die Erfinder haben erkannt, dass mittels der Ausgestaltung der Oberseite des Gehäuses der mobilen Plattform als Ablagefläche der Komfort für das Bedienpersonal und/oder den Patienten erhöht wird. Insbesondere wird der Bewegungsraum der mobilen Plattform nicht durch zusätzliche Aufbauten, welche als Ablagefläche dienen sollen, eingeschränkt. Die mobile Plattform kann flexibel, maximal nah an einen Patienten oder Gegenstand heranfahren bzw. positioniert werden, ohne durch einen Aufbau, welcher als Ablagefläche dienen soll, eingeschränkt zu werden.

In Ausgestaltungen der Erfindung ist das Fahrwerk der mobilen Plattform omnidirektional ausgebildet. Omnidirektional bedeutet, dass die mobile Plattform mit dem Fahrwerk in jede Richtung unabhängig von der Orientierung der mobilen Plattform bewegbar ist.

Das omnidirektionale Fahrwerk kann mindestens eine Rolle umfassen, die in jede Richtung abrollbar ist. Insbesondere kann die mindestens eine Rolle kugelförmig ausgebildet sein.

Alternativ kann das Fahrwerk mindestens ein Rad umfassen. Das mindestens eine Rad ist vorteilhafterweise mit einem Drehgelenk mit dem Gehäuse der mobilen Plattform verbunden. Vorteilhafterweise ist das Drehgelenk drehbar um eine vertikale Achse ausgebildet.

Vorteilhafterweise umfasst das Fahrwerk mindestens vier Rollen oder Räder. Vorteilhafterweise sind die vier Rollen oder Räder im Quadrat oder Rechteck angeordnet.

Alternativ kann das Fahrwerk als Luftkissen ausgebildet sein.

Die Erfinder haben erkannt, dass mittels eines omnidirektionalen Fahrwerkes die Bewegung der mobilen Plattform maximal flexibel ausgestaltet ist. Durch das omnidirektionale Fahrwerk ist kein Rangieren wie bei einem Auto notwendig, wenn die mobile Plattform in eine bestimmte Position verfahren werden soll, da die mobile Plattform mittels des omnidirektionalen Fahrwerkes jederzeit in jede Richtung bewegt werden kann. Die Erfinder haben erkannt, dass dies zu Zeiteinsparungen führt und dass der Platzbedarf der mobilen Plattform geringer ist, da kein Platz zum Rangieren der mobilen Plattform bereitgestellt werden muss. Zudem haben die Erfinder erkannt, dass durch das omnidirektionale Fahrwerk ein Arbeitsraum der mobilen Plattform vergrößert werden kann. Der Arbeitsraum umfasst alle Arbeitspositionen des ersten und/oder dritten medizinischen Gerätes. Insbesondere ist ein flexibles Verfahren in jede Richtung vor, nach oder während einer Untersuchung mittels des omnidirektionalen Fahrwerkes möglich. Dies ermöglich das Abfahren beliebiger Trajektorien durch das omnidirektionale Fahrwerk.

Nach einem weiteren Aspekt betrifft die Erfindung ein System, welches eine Mehrzahl erfindungsgemäßer mobiler Plattformen umfasst. Die Mehrzahl der erfindungsgemäßen mobilen Plattformen ist untereinander koordiniert. Insbesondere kann eine mobile Plattform aus der Mehrzahl der mobilen Plattformen relativ zu der Position oder Bewegung einer anderen mobilen Plattform aus der Mehrzahl der mobilen Plattformen verfahren werden.

Vorteilhafterweise kennt die eine mobile Plattform die Position der anderen mobilen Plattform, zu jedem Zeitpunkt. Alternativ fragt die eine mobile Plattform die Position der anderen mobilen Plattform bei Bedarf und/oder regelmäßig ab. Das Abfragen kann direkt zwischen den mobilen Plattformen ausgeführt werden. Alternativ kann das Abfragen über ein zentrales Verteilersystem ausgeführt werden.

Insbesondere kann diese Koordinierung der Mehrzahl der mobilen Plattformen untereinander über die Orientierung der einzelnen mobilen Plattformen im Raum durch den jeweiligen Orientierungs-Sensor ausgebildet sein. Die einzelnen mobilen Plattformen der Mehrzahl der mobilen Plattformen können ihre Position mit dem jeweiligen Orientierungs-Sensor in ihrer Umgebung bestimmen. Insbesondere können die mobilen Plattformen ihre jeweiligen Positionen direkt an eine andere Plattform der Mehrzahl der mobilen Plattformen übermitteln. Alternativ übermitteln die mobilen Plattformen ihre jeweiligen Positionen an ein zentrales Verteilersystem. Das Verteilersystem leitet die Positionen der einzelnen mobilen Plattform der Mehrzahl der mobilen Plattformen an die jeweils anderen mobilen Plattformen der Mehrzahl der mobilen Plattformen weiter.

Die Übertragung der Information über die Positionen der Mehrzahl der mobilen Plattformen kann insbesondere über Funk realisiert sein. Alternativ können die mobilen Plattformen der Mehrzahl der mobilen Plattformen über wenigstens einen Sensor des Sensormoduls miteinander in Kontakt stehen. In Ausführungsbeispielen kann eine mobile Plattform mit wenigstens einem Ultraschallsensor den Abstand zu einer anderen mobilen Plattform aus der Mehrzahl der mobilen Plattformen bestimmen.

Die Koordinierung der mobilen Plattformen untereinander erleichtert vorteilhafterweise das Nachführen eines ersten medizinischen Gerätes auf einer mobilen Plattform aus der Mehrzahl der mobilen Plattformen einem zweiten medizinischen Gerät auf einer anderen mobilen Plattform aus der Mehrzahl der mobilen Plattformen. Das bedeutet, dass das erste medizinische Gerät mit einer Haltevorrichtung an einer mobilen Plattform befestigt ist und das zweite medizinische Gerät mit einer anderen Haltevorrichtung an einer anderen mobilen Plattform befestigt ist. Insbesondere kann das erste dem zweiten oder das zweite dem ersten medizinischen Gerät nachgeführt sein.

Vorteilhafterweise umfasst die Koordinierung der Mehrzahl der mobilen Plattformen auch ein Austauschen über die Arbeitspositionen der an den einzelnen mobilen Plattformen befestigten medizinischen Geräten in Abhängigkeit von der jeweiligen Haltevorrichtung. Somit wird das Nachführen eines ersten medizinischen Gerätes auf einer mobilen Plattform aus der Mehrzahl der mobilen Plattformen einem zweiten medizinischen Gerät auf einer anderen mobilen Plattform aus der Mehrzahl der mobilen Plattformen mit der jeweiligen Haltevorrichtung und/oder mit dem jeweiligen Fahrwerk vereinfacht bzw. ermöglicht.

In Ausführungsbeispielen können die mobilen Plattformen der Mehrzahl der mobilen Plattformen weitere Informationen insbesondere über Funk austauschen. Insbesondere können die mobilen Plattformen Informationen darüber austauschen, welches medizinische Gerät auf der jeweiligen mobilen Plattform aus der Mehrzahl der mobilen Plattformen montiert ist. Insbesondere können die mobilen Plattformen Informationen über den Belegungsplan bzw. Benutzungsplan jeder einzelnen mobilen Plattform aus der Mehrzahl der mobilen Plattformen austauschen. Der Belegungsplan kann dabei Informationen umfassen, wann die jeweilige mobile Plattform wo benötigt wird und wann sie freie Zeiten hat und/oder wann sie ihr Batteriemodul aufladen muss etc. Auf diese Weise kann die Einsatzzeit der Mehrzahl der mobilen Plattformen optimal ausgeschöpft werden. Die Einsatzzeit der mobilen Plattformen ist die Zeit, die eine mobile Plattform für medizinische Untersuchungen, medizinische Eingriffe und/oder Assistenzfunktionen genutzt werden kann. Zudem muss beispielsweise nicht für jede mobile Plattform der Mehrzahl der mobilen Plattformen eine Ladestation vorgesehen sein. Die mehreren mobilen Plattformen können untereinander ihren Ladestatus abgleichen und die Ladezeiten optimiert bestimmen.

Das Austauschen von Informationen zwischen den mobilen Plattformen wird als Kommunikationsstruktur bezeichnet.

Die Erfinder haben erkannt, dass eine Kommunikationsstruktur zwischen einer Mehrzahl an erfindungsgemäßen mobilen Plattformen, die Durchführung von medizinischen Untersuchungen bzw. medizinischen Eingriffen bzw. Assistenzfunktionen, bei welchen die räumliche Koordination zwischen zwei oder mehr mobilen Plattformen notwendig ist, optimieren kann. Außerdem kann auf diese Weise der gesamte, durch die Mehrzahl an mobilen Plattformen unterstützte Arbeitsablauf in einer Klinik, einer klinischen Abteilung oder einer Praxis, optimiert werden.

In Ausgestaltungen der Erfindung umfasst das System mindestens eine immobile Plattform ohne Fahrwerk, welche mit der Mehrzahl der erfindungsgemäßen mobilen Plattformen koordiniert ist.

Eine immobile Plattform kann wie die mobile Plattform mindestens eine Haltevorrichtung zum Führen eines medizinischen Gerätes umfassen. Diese mindestens eine Haltevorrichtung kann derart ausgebildet sein, dass sie ein medizinisches Gerät innerhalb eines Verstellbereiches in eine Arbeitsposition positionieren kann. Insbesondere ist die immobile Plattform nicht frei in ihrer Umgebung verfahrbar. Vorteilhafterweise ist die Haltevorrichtung der mobilen Plattform an einer Decke, einem Boden oder einer Wand der Umgebung der immobilen Plattform befestigt. Die Umgebung der immobilen Plattform kann beispielsweise eine Radiologie, ein Operationssaal etc. sein. Insbesondere kann die mindestens eine Haltevorrichtung der immobilen Plattform an einem System aus wenigstens einem Führungselement verfahrbar sein. Vorteilhafterweise ist das bzw. die Führungselemente als Schienen ausgebildet. Vorteilhafterweise sind die Schienen an der Decke, der Wand oder dem Boden angeordnet. Vorteilhafterweise ist die mindestens eine Haltevorrichtung entlang der Schienen in mindestens einer Dimension verfahrbar bzw. positionierbar.

Die Mehrzahl der mobilen Plattformen kann mit der mindestens einen immobilen Plattform, wie oben für die Mehrzahl an mobilen Plattformen beschrieben, Informationen austauschen und ist mit der immobilen Plattform koordiniert. Auf diese Weise kann die Bewegung bzw. Position eines ersten medizinischen Gerätes auf einer mobilen Plattform aus der Mehrzahl der mobilen Plattformen der Bewegung bzw. Position eines zweiten medizinischen Gerätes auf der immobilen Plattform nachgeführt werden. Insbesondere kann die Bewegung bzw. Position eines zweiten medizinischen Gerätes auf der immobilen Plattform der Bewegung bzw. Position eines ersten medizinischen Gerätes auf einer mobilen Plattform aus der Mehrzahl der mobilen Plattformen nachgeführt sein.

An der mindestens einen Haltevorrichtung der immobilen Plattform kann insbesondere eine Röntgenröhre befestigt sein. Die Röntgenröhre kann innerhalb des Verstellbereiches der mindestens einen Haltevorrichtung der immobilen Plattform in eine Arbeitsposition positioniert werden und/oder eine Trajektorie umfassend eine Mehrzahl an Arbeitspositionen abfahren. Vorteilhafterweise umfasst eine mobile Plattform aus der Mehrzahl der mobilen Plattformen einen Röntgendetektor. Insbesondere kann die Bewegung bzw. Position des Röntgendetektors auf der mobilen Plattform der Bewegung bzw. Position der Röntgenröhre auf der immobilen Plattform nachgeführt sein. Alternativ kann die Bewegung bzw. Position der Röntgenröhre der Bewegung bzw. Position des Röntgendetektors nachgeführt sein.

Die Erfinder haben erkannt, dass das System umfassend mindestens eine mobile Plattform und mindestens eine immobile Plattform vorteilhaft ist, da es bereits immobile Plattformen in medizinischen Einrichtungen wie Krankenhäusern und/oder Arztpraxen gibt, welche mit der Mehrzahl an mobilen Plattformen vorteilhafterweise koordiniert sind. Insbesondere gibt es medizinische Geräte, die beispielsweise auf Grund des hohen Energiebedarfs nicht auf mobilen Plattformen befestigt werden können, da sie nicht über ein Batteriemodul betrieben werden können. Insbesondere ist eine Koordinierung derartiger medizinischer Geräte auf immobilen Plattformen mit medizinischen Geräten auf mobilen Plattformen vorteilhaft.

Die oben beschriebenen Eigenschaften, Merkmale und Vorteile dieser Erfindung werden klarer und verständlicher im Zusammenhang mit folgenden Figuren und ihren Beschreibungen. Dabei sollen die Figuren und Beschreibungen die Erfindung und ihre Ausführungsformen in keiner Weise einschränken.

In verschiedenen Figuren sind gleiche Komponenten mit korrespondierenden Bezugszeichen versehen. Die Figuren sind in der Regel nicht maßstabsgetreu.

Es zeigen:
- Figur 1: eine Ansicht eines Ausführungsbeispiels einer nicht beanspruchten mobilen Plattform zur automatisierten Durchführung von Ultraschalluntersuchungen,
- Figur 2: eine Ansicht eines Ausführungsbeispiels einer nicht beanspruchten mobilen Plattform zur Durchführung von Assistenzaufgaben,
- Figur 3: eine Ansicht eines Ausführungsbeispiels einer nicht beanspruchten mobilen Plattform umfassend zwei medizinische Geräte an einer teilweise gemeinsamen Haltevorrichtung,
- Figur 4: eine Ansicht eines Ausführungsbeispiels einer nicht beanspruchten mobilen Plattform umfassend drei medizinische Geräte an einer teilweise gemeinsamen Haltevorrichtung,
- Figur 5: eine Ansicht eines Ausführungsbeispiels eines Systems bestehend aus zwei erfindungsgemäßen mobilen Plattformen zum automatisierten Ausführen einer Röntgen-aufnahme,
- Figur 6: eine Ansicht eines Ausführungsbeispiels eines Systems bestehend aus zwei erfindungsgemäßen mobilen Plattformen, wobei die mobilen Plattformen in ihrer Umgebung orientiert sind,
- Figur 7: eine Ansicht eines Ausführungsbeispiels eines Systems bestehend aus einer mobilen und einer immobilen Plattform zum Durchführen einer Röntgenuntersuchung an einem Patienten,
- Figur 8: eine Ansicht des Ausführungsbeispiels des Systems gemäß Figur 7 aus einer alternativen Perspektive.

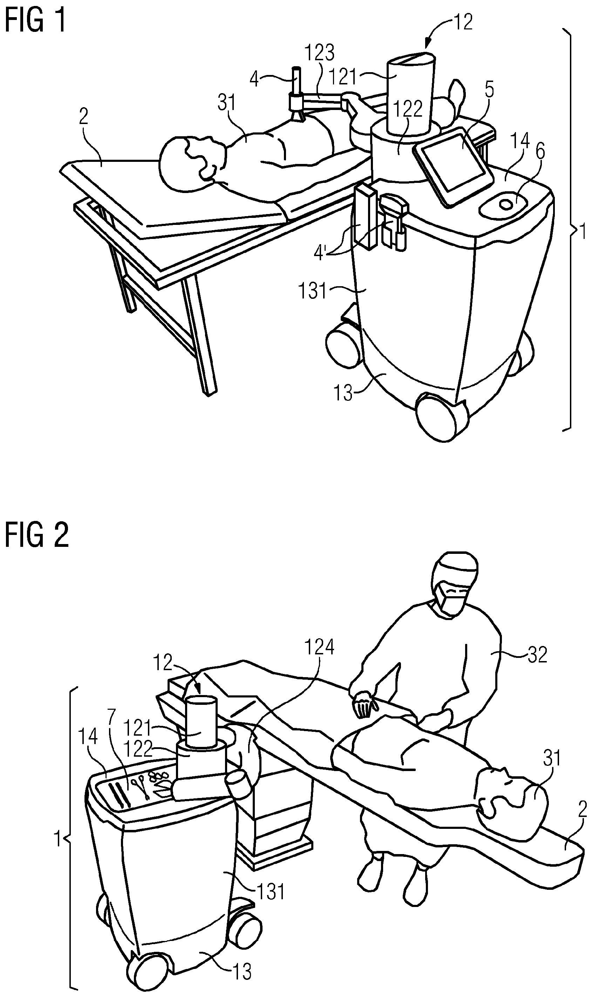

**Figur 1** zeigt eine Ansicht eines Ausführungsbeispiels einer nicht beanspruchten mobilen Plattform 1 zur automatisierten Durchführung von Ultraschalluntersuchungen. Die mobile Plattform 1 umfasst eine Haltevorrichtung 12 und ein omnidirektional fahrbares Fahrwerk 13. Das Gehäuse 131 der mobilen Plattform 1 ist derart ausgebildet, dass es eine Ablagefläche 14 auf Tischhöhe umfasst. Die Haltevorrichtung 12 umfasst eine Hubvorrichtung 121, die über die Ablagefläche 14 hinausragt, eine an der Hubvorrichtung 121 befestigte Drehvorrichtung 122, die oberhalb der Ablagefläche 14 drehbar um die Haltevorrichtung 121 ausgebildet ist und einen an der Drehvorrichtung 122 befestigten robotischen Aktuator 123. Je nach Applikation können die Hubvorrichtung 121, die Drehvorrichtung 122, der robotische Aktuator 123 und/oder in alternativen Ausführungen die Manipulationsvorrichtung 124 derart ausgebildet sein, dass sie selbsttätig den gesamten Verstellbereich am Patienten von Kopf bis Fuß abdecken können. Dies gilt für alle in den folgenden Figuren gezeigten Ausführungsbeispiele. Das bodengestützte omnidirektionale Fahrwerk 13 und die Haltevorrichtung 12 sind im selben räumlichen Koordinatensystem orientiert. Insbesondere können somit zueinander koordinierte Bewegungen von Fahrwerk 13 und Haltevorrichtung 12 ausgeführt werden. Dies gilt für alle in den folgenden Figuren gezeigten Ausführungsbeispiele. An dem robotischen Aktuator 123 ist ein Ultraschallkopf 4 befestigt. Weitere Ultraschallköpfe 4' zum Wechseln sind an der Seite der Ablagefläche 14 der mobilen Plattform 1 angeordnet. In alternativen Ausführungen kann die Haltevorrichtung 12 statt eines robotischen Aktuators 123 eine Manipulationsvorrichtung 124 umfassen. Die Manipulationsvorrichtung 124 kann den für eine entsprechende Untersuchung geeigneten Ultraschallkopf 4, 4' automatisiert greifen. Im Falle eines robotischen Aktuators 123, muss ein Bediener des Gerätes den entsprechenden Ultraschallkopf 4, 4' an dem robotischen Aktuator 123 befestigen. Auf der Ablagefläche 14 der mobilen Plattform 1 ist ein Monitor 5 zum Anzeigen der Bilddaten der Ultraschalluntersuchung angeordnet. Insbesondere ist auf der Ablagefläche 14 der mobilen Plattform 1 eine Eingabeeinrichtung 6 für das Ultraschallgerät angeordnet, mit welcher geeignete Parameter für die Untersuchung und die Anzeige einstellbar sind. Die mobile Plattform 1 steht vor einer Patientenliege 2, auf welcher ein Patient 31 liegt. Mit dem Patientenerkennungs-Sensor und der Körperregionserkennung kann die mobile Plattform 1 den Bauchbereich des Patienten 31 erkennen und automatisiert eine Ultraschalluntersuchung im Bauchbereich durchführen. Mit dem Fahrwerk 13 kann die mobile Plattform 1 jederzeit ihre Position in der Umgebung verändern bzw. anpassen. Insbesondere kann sich die mobile Plattform 1 automatisch zum Durchführen einer Ultraschalluntersuchung positionieren.

**Figur 2** zeigt eine Ansicht eines Ausführungsbeispiels einer nicht beanspruchten mobilen Plattform 1 zur Durchführung von Assistenzaufgaben. Insbesondere ist die mobile Plattform 1 zur Unterstützung des medizinischen Workflows ausgebildet. Die Haltevorrichtung 12 der mobilen Plattform 1 ist ähnlich zu der Haltevorrichtung 12 gemäß Figur 1 ausgebildet. Im Unterschied zu der Haltevorrichtung 12 gemäß Figur 1 umfasst die Haltevorrichtung 12 hier anstelle eines robotischen Aktuators 123 eine Manipulationsvorrichtung 124. Die mobile Plattform 1 ist vor einer Patientenliege 2, auf welcher ein Patient 31 liegt, positioniert. Auf der anderen Seite der Patientenliege 2 steht ein Bediener bzw. Nutzer 32 der mobilen Plattform 1. Der Bediener bzw. Nutzer 32 der mobilen Plattform 1 ist hier ein Arzt oder medizinisches Personal, welches eine Untersuchung am Patienten 31 durchführt. Auf der Ablagefläche 14 der mobilen Plattform 1 ist eine Vielzahl an medizinischen Kleingeräten 7, wie beispielsweise medizinische Instrumente und/oder medizinische Materialien und/oder ein Endoskop und/oder eine Arbeitsleuchte, angeordnet. Die mobile Plattform 1 kann mittels der Manipulationsvorrichtung 124 ein medizinisches Kleingerät 7 von der Ablagefläche 14 aufnehmen und dem Bediener bzw. Nutzer 32 für die Untersuchung und/oder Behandlung des Patienten 31 anreichen.

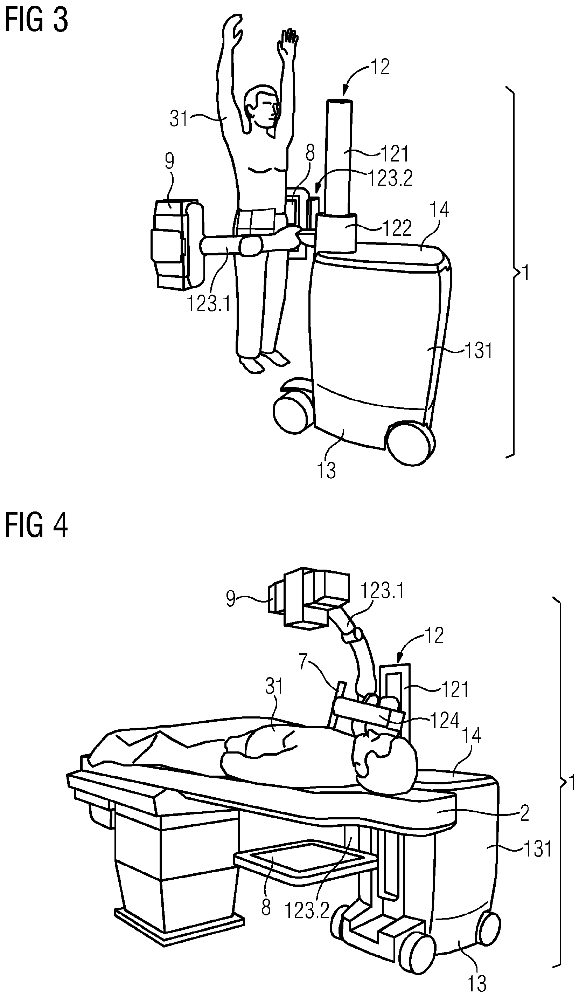

**Figur 3** zeigt eine Ansicht eines Ausführungsbeispiels einer nicht beanspruchten mobilen Plattform 1 umfassend zwei medizinische Geräte 8, 9 an einer teilweise gemeinsamen Haltevorrichtung 12. Die mobile Plattform 1 ist ausgebildet zur automatisierten Aufnahme von Röntgenaufnahmen eines Patienten 31. Die Haltevorrichtung 12 der mobilen Plattform 1 ist ähnlich zu der Haltevorrichtung 12 gemäß Figur 1 ausgebildet. Im Unterschied zu der Haltevorrichtung 12 gemäß Figur 1 umfasst die Haltevorrichtung 12 hier anstelle eines robotischen Aktuators 123 zwei robotische Aktuatoren 123.1, 123.2, die an derselben Drehvorrichtung 122 befestigt sind. Der hintere robotische Aktuator 123.2 ist in der Ansicht in Figur 3 durch andere Komponenten der mobilen Plattform 1 verdeckt. An dem vorderen robotischen Aktuator 123.1 ist eine Röntgenröhre 9 befestigt. An dem hinteren robotischen Aktuator 123.2 ist ein Röntgendetektor 8 befestigt. Der Röntgendetektor 8 und die Röntgenröhre 9 sind horizontal zueinander ausgerichtet. Andere Ausrichtungen, wie beispielsweise eine vertikale Ausrichtung der Röntgenröhre 9 und des Röntgendetektors 8 sind mit den robotischen Aktuatoren 123.1, 123.2 einstellbar. In dem gezeigten Ausführungsbeispiel ist ein Patient 31 zwischen dem Röntgendetektor 8 und der Röntgenröhre 9 positioniert. Für das Abfahren einer Trajektorie, welche einen kleinen Winkelbereich von rund 10° um einen Patienten umfasst, kann die mobile Plattform 1 den Röntgendetektor 8 und die Röntgenröhre 9 mittels der Drehvorrichtung 122 um den Patienten 31 herumdrehen. Für das Abfahren einer Trajektorie, die einen größeren Winkelbereich als 10° um den Patienten abdeckt, kann die komplette mobile Plattform 1 mit dem omnidirektional fahrbaren Fahrwerk 13 um den Patienten 31 herumgefahren werden, um Röntgenaufnahmen aus verschiedenen Winkeln aufzunehmen. Insbesondere kann auf diese Weise eine kreisrunde Trajektorie abgefahren werden, deren Zentrum der Patient 31 ist. Zudem kann die Anordnung des Röntgendetektors 8 und der Röntgenröhre 9 mit der Hubvorrichtung 121 in der Höhe verstellt werden. Dies ist insbesondere notwendig, wenn ein Bereich des Patienten 31 aufgenommen werden soll, welcher größer als die vertikale Ausdehnung des Röntgendetektors 8 ist. Durch die Höhenverstellbarkeit des Röntgendetektors 8 und der Röntgenröhre 9 kann ein vertikaler Scan des Patienten 31 erstellt werden. Insbesondere ist die Höhenverstellbarkeit des Röntgendetektors 8 und der Röntgenröhre 9 zur Anpassung der Aufnahmehöhe an die Patientengröße vorteilhaft. Die Einstellungen der Höhe und des Winkels, in welchen die Röntgenaufnahme des Patienten 31 aufgenommen werden soll, können mit dem Patientenerkennungs-Sensor und der Körperregionserkennung automatisiert von der mobilen Plattform 1 bestimmt werden. Insbesondere kann die Trajektorie, die beispielsweise für eine Mehrzahl an Aufnahmen aus verschiedenen Winkeln um den Patienten 31 herum abgefahren werden muss, mit dem Patientenerkennungs-Sensor und der Körperregionserkennung bestimmt werden. Alternativ kann die Trajektorie voreingestellt sein, beispielsweise, wenn alle zu untersuchenden Patienten an einer vordefinierten Position positioniert sind und die mobile Plattform 1 die Trajektorie relativ zur Umgebung abfahren kann. Insbesondere kann durch ein gleichzeitiges Verfahren der mobilen Plattform 1 mit dem Fahrwerk 13 und ein Bewegen der Röntgenröhre 9 und des Röntgendetektors 8 eine beliebige Trajektorie um den Patienten 31 abgefahren werden.

Das Bestimmen der geeigneten Position der mobilen Plattform 1 für eine medizinische Untersuchung mit dem Patientenerkennungs-Sensor bietet den Vorteil, dass Untersuchungen direkt im Patientenzimmer durchgeführt werden können und der Patient 31 nicht mehr zur Untersuchung transportiert werden muss. Der Patient 31 muss zur Untersuchung keine definierte Position mit der mobilen Plattform 1 einnehmen. Insbesondere ist kein Bediener 32 vor Ort notwendig, da die mobile Plattform 1 mit den Orientierungs- und Kollisions-Sensoren im Raum orientiert ist und Untersuchungen mit Hilfe des Patientenerkennungs-Sensors automatisiert durchführen kann.

**Figur 4** zeigt eine Ansicht eines Ausführungsbeispiels einer nicht beanspruchten mobilen Plattform 1 umfassend drei medizinische Geräte 7, 8, 9, die an einer teilweise gemeinsamen Haltevorrichtung 12 angeordnet sind. Die mobile Plattform 1 ist zur Durchführung medizinischer Eingriffe ausgebildet. Die gezeigte mobile Plattform 1 umfasst eine Haltevorrichtung 12 und ein omnidirektional fahrbares Fahrwerk 13. Das Gehäuse 131 der mobilen Plattform 1 ist derart ausgebildet, dass es eine Ablagefläche 14 auf Tischhöhe umfasst. Die Haltevorrichtung 12 umfasst eine Hubvorrichtung 121, die über die Ablagefläche 14 hinausragt, zwei robotische Aktuatoren 123.1, 123.2 und eine Manipulationsvorrichtung 124. Die robotischen Aktuatoren 123.1, 123.2 und die Manipulationsvorrichtung 124 sind an derselben Hubvorrichtung 121 befestigt. An dem unteren robotischen Aktuator 123.2 ist ein Röntgendetektor 8 befestigt. Am oberen robotischen Aktuator 123.1 ist eine Röntgenröhre 9 befestigt. Mit der der Manipulationsvorrichtung 124 kann ein medizinisches Kleingerät 7 gegriffen werden. Die mobile Plattform 1 ist vor einer Patientenliege 2 positioniert, auf welcher ein Patient 31 liegt. Über die Referenzpunkte in der Umgebung und/oder an der Patientenliege 2 ist die mobile Plattform 1 derart im Raum orientiert, dass sie eine Position an der Patientenliege 2 gezielt ansteuern kann. Mit dem Patientenerkennungs-Sensor und der Körperregionserkennung kann die mobile Plattform 1 die Körperregion des Patienten 31 erkennen, in welcher die Behandlung durchgeführt werden soll. Die mobile Plattform 1 nimmt mit dem Röntgendetektor 8 und der Röntgenröhre 9 Röntgenbilder der entsprechenden Körperregion des Patienten 31 auf. Mit diesen Bilddaten aus der Röntgenaufnahme kann die mobile Plattform 1 die Bewegung bzw. das Führen des medizinischen Kleingerätes 7 durch die Manipulationsvorrichtung 124 koordinieren. Die Bilddaten dienen somit der Koordination des medizinischen Kleingerätes 7 im Verhältnis zur mobilen Plattform 1 und ermöglicht so insbesondere eine Feinkoordinierung der Bewegung der Manipulationsvorrichtung 124 anhand der Bilddaten. Auf diese Weise können medizinische Eingriffe automatisiert mittels der mobilen Plattform 1 durchgeführt werden. Insbesondere kann derart die Position des medizinischen Gerätes 7, 8, 9 an die Bewegung des Patienten 31 bzw. der Organe des Patienten 31 angepasst werden.

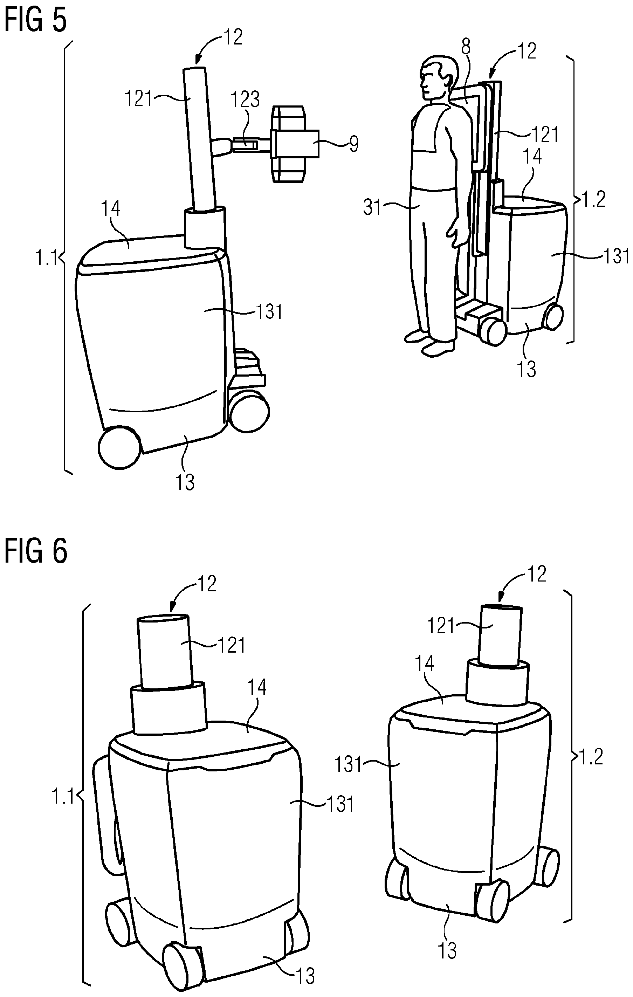

**Figur 5** zeigt eine Ansicht eines Ausführungsbeispiels eines Systems bestehend aus zwei erfindungsgemäßen mobilen Plattformen 1.1, 1.2, die zum automatisierten Ausführen einer Röntgenaufnahme ausgebildet sind. In alternativen Ausführungsformen kann das System mehr als zwei mobile Plattformen umfassen. Die mobilen Plattformen 1.1, 1.2 umfassen jeweils ein omnidirektional fahrbares Fahrwerk 13 und ein Gehäuse 131, das derart ausgebildet ist, dass es eine Ablagefläche 14 auf Tischhöhe umfasst.

Die erste mobile Plattform 1.1 umfasst außerdem eine Haltevorrichtung 12, welche eine Hubvorrichtung 121 und einen an der Hubvorrichtung 121 befestigten robotischen Aktuator 123 umfasst. An dem robotischen Aktuator 123 ist eine Röntgenröhre 9 befestigt.

Die zweite mobile Plattform 1 umfasst außerdem eine Haltevorrichtung 12, welche eine Hubvorrichtung 121 umfasst. An der Hubvorrichtung 121 ist ein Röntgendetektor 8 befestigt.

Die beiden mobilen Plattformen 1.1, 1.2 sind zueinander in der Umgebung koordiniert. Außerdem können die mobilen Plattformen 1.1, 1.2 mit den jeweiligen Patientenerkennungs-Sensoren und der Körperregionserkennung einen Patienten 31 erkennen und die Röntgenröhre 9 und den Röntgendetektor 8 automatisch derart positionieren, dass eine Röntgenaufnahme der von einem Bediener voreingestellten Körperregion eines Patienten 31 aufgenommen werden kann. Dabei kann die Positionierung der Röntgenröhre 9 und des Röntgendetektors 8 für Patienten 31 jeder Körpergröße und für eine beliebige Position des Patienten 31 vorgenommen werden, da das System der mobilen Plattformen 1.1, 1.2 mit Orientierungs-, Kollisions- und Patientenerkennungs-Sensor untereinander und in der Umgebung koordiniert ist. Die beiden mobilen Plattformen 1.1, 1.2 können zueinander koordiniert Trajektorien zur Aufnahme einer Mehrzahl an Röntgenaufnahmen beispielsweise ähnlich wie bei eine C-Bogen abfahren. Dabei kann die Trajektorie in Ausführungen im Vorhinein von einem Bediener 32 vorgegeben sein. Alternativ können die mobilen Plattformen 1.1, 1.2 die Trajektorie mit den Sensoren des jeweiligen Sensormoduls bestimmen. Die Trajektorie kann relativ zu der Patientenposition oder, wenn alle Patienten 31 immer in derselben Position positioniert sind, relativ zu der Umgebung abfahren werden. Die Trajektorie kann mit den jeweiligen Fahrwerken 13 abgefahren werden. Alterativ oder zusätzlich kann die Trajektorie eine Trajektorie der Röntgenröhre 9 und des Röntgendetektors 8 sein. Diese Trajektorie kann mit der jeweiligen Haltevorrichtung 12 abgefahren werden. In Ausführungen kann die Trajektorie mit den Fahrwerken 13 und den Haltevorrichtungen 12 in Kombination abgefahren werden. Insbesondere kann die Trajektorie nur einer der beiden mobilen Plattformen 1.1, 1.2 vorgegeben sein bzw. von einer der beiden mobilen Plattformen 1.1, 1.2 bestimmt werden. Die andere mobile Plattform 1.1, 1.2 kann der Bewegung der einen mobilen Plattform 1.1, 1.2 nachgeführt werden. Dafür kann die andere mobile Plattform 1,1, 1.2 die Bewegung der einen mobilen Plattform 1.1, 1.2 mit den Sensoren ihrer Sensoreinheit detektieren. Alternativ können die beiden mobilen Plattformen 1.1, 1.2 ihre Daten über ihre jeweilige Position in der Umgebung und der Position des medizinischen Gerätes wie der Röntgenröhre 9 oder dem Röntgendetektor 8 an der jeweiligen Haltevorrichtung 12 der mobilen Plattform 1.1, 1.2 austauschen. Das Austauschen der Positionen kann direkt zwischen den beiden mobilen Plattformen 1.1, 1.2 oder über ein zentrales Verteilersystem ausgeführt werden.

Für solche Anwendungen, beispielsweise in der Röntgenbildgebung, bei welcher Röntgenröhre 9 und Röntgendetektor 8 auf zwei mobilen Plattformen 1.1, 1.2 angeordnet sind, wie in dem in dieser Figur gezeigten Ausführungsbeispiel oder, wenn an Haltevorrichtungen 12 befestigte medizinische Geräte 7, 8, 9 bei einem medizinischen Eingriff kollaborieren, können sich zwei oder mehrere mobile Plattformen 1.1, 1.2 mit Submillimeter-Genauigkeit zueinander positionieren. Dies kann in Ausführungen durch die direkte, über Funk übertragene Abstands- und/oder Positionsinformationen zwischen den mobilen Plattformen 1.1, 1.2 realisiert werden. Alternativ kann die Positionierung der mobilen Plattform 1.1, 1.2 über Referenzpunkte in der Umgebung der mobilen Plattformen 1.1, 1.2 realisiert werden. Die Referenzpunkte werden von den jeweiligen Orientierungs-Sensoren der mobilen Plattformen 1.1, 1.2 erfasst. Die mobilen Plattformen 1.1, 1.2 können somit über die Referenzpunkte in derselben Umgebung koordiniert sein. Somit sind die mobilen Plattformen 1.1, 1.2 indirekt auch untereinander koordiniert.

**Figur 6** zeigt eine Ansicht eines Ausführungsbeispiels eines Systems bestehend aus zwei erfindungsgemäßen mobilen Plattformen 1.1, 1.2, wobei die mobilen Plattformen 1.1, 1.2 in ihrer Umgebung orientiert sind. In alternativen Ausführungsformen kann das System mehr als zwei mobile Plattformen umfassen. Die zwei mobilen Plattformen 1.1, 1.2 können koordiniert in ihrer Umgebung verfahren. Beide mobilen Plattformen 1.1, 1.2 umfassen jeweils ein omnidirektional fahrbares Fahrwerk 13 und jeweils ein Gehäuse 131. Die Gehäuse 131 sind derart ausgebildet, dass sie jeweils eine Ablagefläche 14 auf Tischhöhe ausbilden. Die Haltevorrichtungen 12 der beiden mobilen Plattformen 1.1, 1.2 umfassen jeweils eine Hubvorrichtung 121, welche teleskopartig ausgebildet ist. Das bedeutet die Hubvorrichtungen 121 sind platzsparend in der Höhe ineinanderfahrbar bzw. -schiebbar. Für das Verfahren in der Umgebung, beispielsweise für das Wechseln von Räumen in einer Klinik können die Hubvorrichtungen 121 auf diese Weise platzsparend ineinandergeschoben werden.

Die beiden mobilen Plattformen 1.1, 1.2 sind zueinander koordiniert. Außerdem ist jede der mobilen Plattformen 1.1, 1.2 in der Umgebung koordiniert. Auf diese Weise können sich die mobilen Plattformen 1.1, 1.2 automatisiert in ihrer Umgebung beispielsweise einer Klinik oder einer Klinikabteilung oder einer Arztpraxis positionieren. Das Positionieren ist abhängig von dem Ort, an dem sie für eine medizinische Untersuchung oder einen medizinischen Eingriff oder Assistenzfunktionen benötigt werden. Außerdem können die mobilen Plattformen 1.1, 1.2 Transportfunktionen übernehmen und Material und/oder Geräte in einen Raum bringen, in welchem dieses benötigt wird. Das Material und die Geräte können insbesondere ein medizinisches Kleingerät 7 wie ein Endoskop, eine Arbeitsleuchte, ein medizinisches Instrument oder medizinisches Material sein.

Das Sensormodul mit Sensoren zur Positionsbestimmung bzw. Orientierung im Raum (mit Odometrie, Tracking und Vermessung im Raum -optisch, inertial, akustisch oder funktechnisch) sowie mit Sensoren zur Erkennung und Vermeidung von Kollisionen ermöglicht die assistierte, autonome oder teilautonome Bewegung der mobilen Plattformen 1.1, 1.2 innerhalb der Umgebung der mobilen Plattformen 1,1, 1.2, beispielsweise innerhalb eines Arbeitsraums oder in ganzen Gebäuden. Die Positionsgenauigkeit ist je nach Anforderung zentimetergenau bei Transportfahrten zwischen Arbeitsräumen bzw. Räumen oder submillimetergenau bei präzisen Bildgebungs- oder Therapieanwendungen.

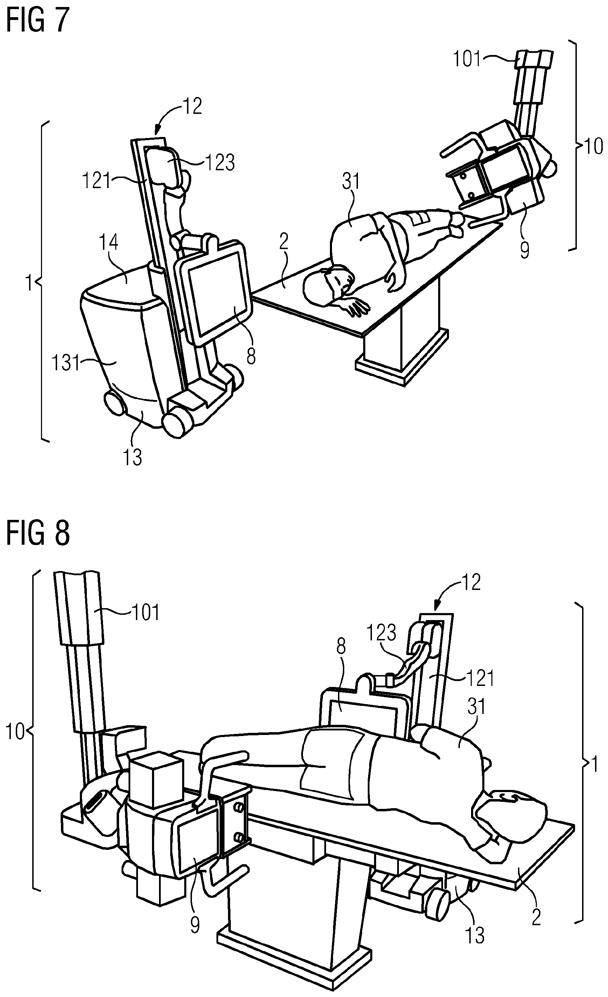

**Figur** 7 zeigt eine Ansicht eines Ausführungsbeispiels eines Systems bestehend aus einer mobilen Plattform 1 und einer immobilen Plattform 10 zum Durchführen einer Röntgenuntersuchung an einem Patienten 31. In alternativen Ausführungsformen kann das System mehr als eine mobile Plattform und/oder mehr als eine immobile Plattform umfassen. Die mobile Plattform 1 umfasst ein omnidirektional fahrbares Fahrwerk 13 und ein Gehäuse 131. Das Gehäuse 131 ist derart ausgebildet, dass es eine Ablagefläche 14 auf Tischhöhe bildet. Die mobile Plattform 1 umfasst außerdem eine Haltevorrichtung 12, welche eine Hubvorrichtung 121 und einen an der Hubvorrichtung 121 befestigten robotischen Aktuator 123 umfasst. An dem robotischen Aktuator 123 ist ein Röntgendetektor 8 befestigt.

Die immobile Plattform 10 umfasst eine Haltevorrichtung 101, welche in diesem Ausführungsbeispiel an der Decke befestigt ist. Alternativ kann die Haltevorrichtung 101 der immobilen Plattform 10 auch am Boden oder an einer Wand des Raumes, in dem sich die immobile Plattform 10 befindet, befestigt sein. Die Haltevorrichtung 101 der mobilen Plattform 10 entspricht in diesem Ausführungsbeispiel einem robotischen Aktuator 123. An der Haltevorrichtung 101 der immobilen Plattform 10 ist eine Röntgenröhre 9 befestigt. Die mobile Plattform 1 und die immobile Plattform 10 sind zueinander koordiniert. Insbesondere sind darüber auch die Röntgenröhre 9 der immobilen Plattform 10 und der Röntgendetektor 8 der mobilen Plattform 1 zueinander koordiniert. In der Umgebung der immobilen Plattform 10 ist ein Patiententisch 2 positioniert, sodass Röntgenaufnahmen von einem auf dem Patiententisch 2 liegenden Patienten 31 mit der Röntgenröhre 9 und dem Röntgendetektor 8 aufgenommen werden können. Der Röntgendetektor 8 der mobilen Plattform 1 wird der Bewegung der Röntgenröhre 9 der immobilen Plattform 10 derart nachgeführt, dass der Röntgendetektor 8 die Röntgenaufnahmen entsprechende der Ausrichtung der Röntgenröhre 9 zum Patienten 31 aufnehmen kann. Somit kann durch gleichzeitiges oder einzelnes Bewegen der mobilen Plattform 1 durch das Fahrwerk 13, des Röntgendetektors 8 mit der Haltevorrichtung 12 und der Röntgenröhre 9 mit der Haltevorrichtung 101 eine beliebige Trajektorie zur Aufnahme von Röntgenbildern abgefahren werden.

**Figur** 8 zeigt eine Ansicht des Ausführungsbeispiels des Systems gemäß Figur 7 aus einer alternativen Perspektive und mit einer alternativen Positionierung der Röntgenröhre 9 und des Röntgendetektors 8. Die Röntgenröhre 9 und der Röntgendetektor 8 sind derart positioniert, dass sie eine Röntgenaufnahme einer Körperregion des Patienten 31 aufnehmen können. Die Positionierung der Röntgenröhre 9 kann dabei anhand der Koordinierung der immobilen Plattform 10 im Raum durchgeführt werden. Die immobile Plattform 10 kann dafür analog zu der mobilen Plattform 1 in der Umgebung koordiniert sein. Alternativ kann die Positionierung der Röntgenröhre 9 anhand der Koordinierung der immobilen Plattform 10 über die von einem Orientierungs-Sensor erfassten Referenzpunkte an der Patientenliege 2 und/oder mit einem Patientenerkennungs-Sensor und der Körperregionserkennung durchgeführt werden. Die Position bzw. Bewegung des Röntgendetektors 8 kann der Position bzw. Bewegung der Röntgenröhre 9 nachgeführt sein. Das Nachführen kann analog zu dem Nachführen von medizinischen Geräten zwischen zwei mobilen Plattformen 1.1, 1.2 durchgeführt werden.

Alternativ kann die mobile Plattform 1 in der Umgebung und/ oder an der Patientenliege koordiniert sein. Insbesondere kann dann die Position bzw. Bewegung der Röntgenröhre 9 der Position bzw. Bewegung des Röntgendetektors 8 nachgeführt sein.

Es können somit frei bewegliche/dynamische Anordnungen der Röntgenröhre 9 zum Röntgendetektor 8 eingenommen werden. Insbesondere können so radiologische 2-D- und/oder 3-D-Bilddaten in veränderlichen Posen und Positionen des Patienten 31 aufgenommen werden. Es können statische und dynamische Bildaufnahmen durchgeführt werden.

Obwohl die Erfindung im Detail mit Bezug auf die bevorzugten Ausführungsbeispiele näher illustriert und beschrieben wurde, so ist die Erfindung nicht hierdurch eingeschränkt. Andere Variationen und Kombinationen können vom Fachmann hieraus abgeleitet werden, ohne vom wesentlichen Gedanken der Erfindung abzuweichen.

## Patentansprüche

1. Mobile Plattform (1, 1.1, 1.2) umfassend
ein Fahrwerk (13),
ein Sensormodul und
mindestens eine Haltevorrichtung (12), wobei
- die Haltevorrichtung zum Führen eines ersten medizinischen Gerätes (4, 4', 7, 8, 9) ausgebildet ist,
- die Haltevorrichtung derart ausgestaltet ist, das erste medizinische Gerät innerhalb eines Verstellbereiches in mindestens eine Arbeitsposition zu positionieren,
- das Sensormodul mindestens einen Orientierungs-Sensor umfasst, welcher ausgebildet ist, wenigstens einen in einer Umgebung der mobilen Plattform angeordneten Referenzpunkt zu erfassen und
- das Fahrwerk und/oder die mindestens eine Haltevorrichtung ausgebildet sind, die Arbeitsposition des an der mindestens einen Haltevorrichtung angebrachten ersten medizinischen Gerätes der Bewegung eines zweiten medizinischen Gerätes anzupassen,
wobei das zweite medizinische Gerät von einer zweiten mobilen Plattform oder von einer an einer immobilen Plattform angebrachten, zweiten beweglichen Haltevorrichtung geführt wird,
wobei das zweite medizinische Gerät seine Position an die mobile Plattform übermittelt. **dadurch gekennzeichnet, dass** das Fahrwerk und/oder die Haltevorrichtung ausgebildet sind, das Anpassen der Arbeitsposition des ersten medizinischen Geräts an die Bewegung des zweiten medizinischen Geräts auszuführen, indem das erste medizinische Gerät abhängig von der übermittelten Position des zweiten medizinischen Geräts positioniert wird.

2. Mobile Plattform nach Anspruch 1, wobei das erste medizinische Gerät ein Gerät aus der folgenden Gruppe medizinischer Geräte ist: Röntgendetektor (8), eine Röntgenröhre (9), ein Ultraschallkopf (4, 4'), ein Endoskop, eine Arbeitsleuchte, ein medizinisches Instrument oder medizinisches Material.

3. Mobile Plattform nach einem der vorhergehenden Ansprüche, wobei die mindestens eine Haltevorrichtung eine Hubvorrichtung (121), eine Drehvorrichtung (122), einen robotischen Aktuator (123, 123.1, 123.2) und/oder eine Manipulationsvorrichtung (124) umfasst.

4. Mobile Plattform nach einem der vorhergehenden Ansprüche, wobei das Sensormodul mindestens einen Kollisions-Sensor umfasst, welcher ausgebildet ist, Objekte in der Umgebung der mobilen Plattform zu erkennen.

5. Mobile Plattform nach einem der vorhergehenden Ansprüche, wobei das Sensormodul mindestens einen Patientenerkennungs-Sensor umfasst.

6. Mobile Plattform nach einem der vorhergehenden Ansprüche, umfassend mindestens zwei Haltevorrichtungen, wobei
- die Haltevorrichtungen jeweils zum Führen eines ersten und eines dritten medizinischen Gerätes ausgebildet sind und
- die Haltevorrichtungen derart ausgestaltet sind, jeweils das erste und das dritte medizinische Gerät innerhalb eines Verstellbereiches in mindestens eine Arbeitsposition zu positionieren.

7. Mobile Plattform nach einem der vorhergehenden Ansprüche, wobei die mobile Plattform ein Batteriemodul umfasst.

8. Mobile Plattform nach einem der vorhergehenden Ansprüche umfassend einen Kippschutz.

9. Mobile Plattform nach einem der vorhergehenden Ansprüche umfassend eine Ablagefläche (14).

10. Mobile Plattform nach einem der vorhergehenden Ansprüche, wobei das Fahrwerk omnidirektional ausgebildet ist.

11. System umfassend eine Mehrzahl erfindungsgemäßer mobiler Plattformen nach einem der vorhergehenden Ansprüche, wobei die Mehrzahl der mobilen Plattformen untereinander koordiniert ist.

12. System nach Anspruch 11, wobei das System mindestens eine immobile Plattform (10) ohne Fahrwerk umfasst, welche mit der Mehrzahl der erfindungsgemäßen mobilen Plattformen koordiniert ist.

## Claims

1. Mobile platform (1, 1.1, 1.2) comprising
a chassis (13),
a sensor module and
at least one holding mechanism (12), wherein
- the holding mechanism is designed for guiding a first medical device (4, **4',** 7, 8, 9),
- the holding mechanism is configured in such a way as to position the first medical device in at least one operating position within an adjusting range,
- the sensor module comprises at least one orientation sensor, which is designed to detect at least one reference point arranged in an environment of the mobile platform and
- the chassis and/or the at least one holding mechanism is/are designed to adjust the operating position of the first medical device attached to the at least one holding mechanism to the movement of a second medical device,
wherein the second medical device is guided by a second mobile platform or by a second movable holding mechanism that is attached to an immobile platform,
wherein the second medical device transmits its position to the mobile platform, **characterised in that** the chassis and/or the holding mechanism are embodied to carry out the adjustment of the operating position of the first medical device to the movement of the second medical device, by the first medical device being positioned as a function of the transmitted position of the second medical device.

2. Mobile platform according to claim 1, wherein the first medical device is a device from the following group of medical devices: X-ray detector (8), an X-ray tube (9), an ultrasound head (4, 4'), an endoscope, a work light, a medical instrument or medical material.

3. Mobile platform according to one of the preceding claims, wherein the at least one holding mechanism comprises a lifting device (121), a rotating device (122), a robotic actuator (123, 123.1, 123.2) and/or a manipulation device (124).

4. Mobile platform according to one of the preceding claims, wherein the sensor module comprises at least one collision sensor, which is designed to detect objects in the environment of the mobile platform.

5. Mobile platform according to one of the preceding claims, wherein the sensor module comprises at least one patient detection sensor.

6. Mobile platform according to one of the preceding claims, comprising at least two holding devices, wherein
- the holding devices are each designed for guiding a first and a third medical device and
- the holding devices are configured in such a way as to position the first and the third medical device respectively in at least one operating position within an adjusting range.

7. Mobile platform according to one of the preceding claims, wherein the mobile platform comprises a battery module.

8. Mobile platform according to one of the preceding claims comprising an anti-tilt mechanism.

9. Mobile platform according to one of the preceding claims comprising a shelf space (14).

10. Mobile platform according to one of the preceding claims, wherein the chassis is designed to be omnidirectional.

11. System comprising a plurality of inventive mobile platforms according to one of the preceding claims, wherein the plurality of mobile platforms is coordinated among themselves.

12. System according to claim 11, wherein the system comprises at least one immobile platform (10) without chassis, which is coordinated with the plurality of inventive mobile platforms.

## Revendications

1. Plateforme (1, 1.1, 1.2) mobile comprenant un chariot (13),
un module de capteur et
au moins un dispositif (12) de maintien, dans laquelle
- le dispositif de maintien est constitué pour guider un premier appareil (4, 4', 7, 8, 9) médical,
- le dispositif de maintien est conformé, de manière à mettre le premier appareil médical en au moins une position de travail dans une plage de réglage,
- le module de capteur comprend au moins un capteur d'orientation, qui est constitué pour détecter au moins un point de référence disposé dans un environnement de la plateforme mobile et
- le chariot et/ou le au moins un dispositif de maintien sont constitués pour adapter la position de travail du premier appareil médical monté sur le au moins un dispositif de maintien au déplacement d'un deuxième appareil médical,
dans laquelle le deuxième appareil médical est guidé par une deuxième plateforme mobile ou par un deuxième dispositif de maintien mobile monté sur une plateforme immobile,
dans laquelle le deuxième appareil médical transmet sa position à la plateforme mobile, **caractérisée en ce que** le chariot et/ou le dispositif de maintien sont constitués pour effectuer l'adaptation de la position de travail du premier appareil médical au déplacement du deuxième appareil médical, en mettant le premier appareil médical en position en fonction de la position transmise du deuxième appareil médical.

2. Plateforme mobile suivant la revendication 1, dans laquelle le premier appareil médical est un appareil du groupe suivant des appareils médicaux : détecteur (8) de rayons X, un tube (9) à rayons X, une tête (4, 4') d'ultrason, un endoscope, un éclairage de travail, un instrument médical ou du matériel médical.

3. Plateforme mobile suivant l'une des revendications précédentes, dans laquelle le au moins un dispositif de maintien comprend un dispositif (121) de levage, un dispositif (122) de rotation, un actionneur (123, 123.1, 123.2) robotique et/ou un dispositif (124) de manipulation.

4. Plateforme mobile suivant l'une des revendications précédentes, dans laquelle le module de capteur comprend au moins un capteur de collision, qui est constitué pour détecter des objets dans l'environnement de la plateforme mobile.

5. Plateforme mobile suivant l'une des revendications précédentes, dans laquelle le module de capteur comprend au moins un capteur de détection d'un patient.

6. Plateforme mobile suivant l'une des revendications précédentes, comprenant au moins deux dispositifs de maintien, dans laquelle
- les dispositifs de maintien sont constitués respectivement pour le guidage d'un premier et d'un troisième appareil médical et
- les dispositifs de maintien sont conformés pour mettre respectivement le premier et le troisième appareil médical dans au moins une position de travail dans une plage de réglage.

7. Plateforme mobile suivant l'une des revendications précédentes, dans laquelle la plateforme mobile comprend un module de batterie.

8. Plateforme mobile suivant l'une des revendications précédentes, comprenant une protection vis-à-vis du renversement.

9. Plateforme mobile suivant l'une des revendications précédentes, comprenant une surface (14) de dépôt.

10. Plateforme mobile suivant l'une des revendications précédentes, dans laquelle le chariot est de constitution omnidirectionnelle.

11. Système comprenant une pluralité de plateformes mobiles suivant l'invention, suivant l'une des revendications précédentes, dans lequel la pluralité des plateformes mobiles sont coordonnées entre elles.

12. Système suivant la revendication 11, dans lequel le système comprend au moins une plateforme (10) immobile sans chariot, qui est coordonnée à la pluralité de plateformes mobiles suivant l'invention.
